Europäisches Patentamt

(19) European Patent Office

Office européen des brevets

(11) EP 0 977 596 B1

## (12) EUROPEAN PATENT SPECIFICATION

(45) Date of publication and mention
of the grant of the patent:
**21.01.2004 Bulletin 2004/04**

(21) Application number: **98919321.4**

(22) Date of filing: **27.04.1998**

(51) Int Cl.[7]: **A61K 48/00**

(86) International application number:
**PCT/GB1998/001230**

(87) International publication number:
**WO 1998/048841 (05.11.1998 Gazette 1998/44)**

(54) **USE OF PAPOVAVIRUS CAPSID PROTEIN FOR DELIVERY OF THERAPEUTIC AGENTS TO NEURONAL CELLS**

VERWENDUNG VON PAPOVAVIRUS KAPSID-PROTEINEN ZUR VERABREICHUNG THERAPEUTISCHEN AGENTIEN IN NERVZELLEN

UTILISATION DE PROTEINE CAPSIDIQUES DE PAPOVAVIRUS POUR TRANSFERER DES AGENTS THERAPEUTIQUES DANS LES CELLULES NEURONALES

(84) Designated Contracting States:
**CH DE FR GB IT LI NL**

(30) Priority: **26.04.1997 GB 9708415
29.04.1997 GB 9708632**

(43) Date of publication of application:
**09.02.2000 Bulletin 2000/06**

(73) Proprietor: **RPMS TECHNOLOGY LIMITED**
**London W12 0NN (GB)**

(72) Inventors:
• **GRIFFIN, Beverly, Elayne**
**Royal Postgraduate Med.**
**London W12 0NN (GB)**
• **KRAUZEWICZ, Nina, Sheila**
**Royal Postgraduate Med.**
**London W12 0NN (GB)**

(74) Representative: **Thomas, Philip John Duval**
**Eric Potter Clarkson,**
**Park View House,**
**58 The Ropewalk**
**Nottingham NG1 5DD (GB)**

(56) References cited:
**WO-A-95/19368          WO-A-97/17456**

• **FORSTOVA J ET AL: "POLYOMA VIRUS PSEUDOCAPSIDS AS EFFICIENT CARRIERS OF HETEROLOGOUS DNA INTO MAMMALIAN CELLS" HUMAN GENE THERAPY, vol. 6, no. 3, March 1995, pages 297-306, XP000645479 cited in the application**

**Description**

The present invention relates to the packaging of material for delivery, for example in gene therapy and other situations.

Gene transfer, as a means to effect gene therapy, is becoming increasingly important. Numerous studies have been carried out that seek to harness the inherent ability of viruses to infect eukaryotic cells in order to introduce a selected gene into a suitable recipient host (as reviewed: Anderson, 1992; Morgan and Anderson, 1993; Mulligan, 1993). Adenoviruses have proved particularly attractive for this purpose in that their genome can be manipulated to incorporate up to about 8kbp of foreign DNA and, unlike retroviral vectors, transduced genes can be expressed in non-dividing cells. One particular handicap shared by all the present virus vectors, however, is that they introduce unwanted viral genetic information alongside the gene of interest into the recipient host. The disadvantages of this unwanted viral material remain to be determined fully, but there is a great need for means to carry out improved genetic transfer without introduction of unwanted viral genes into target cells.

Physical, non-viral gene transfer methods such as chemical and mechanical techniques and membrane fusion-mediated transfer via liposomes have been used (Morgan and Anderson, 1993). However, the efficiency of transfer, in terms of expression of the genetic material transferred, is low. In other words, these methods are inefficient at transferring genetic material into cells in a stable manner so that the material is biologically functional.

The empty capsids of papovaviruses such as the mouse polyoma virus have received attention as possible vectors for gene transfer.

Barr *et al*, 1979, first described the use of polyoma empty particles when polyoma DNA and purified empty capsids were incubated in a cell-free system. The DNA of the new particle was protected from the action of pancreatic DNase. Slilaty and Aposhian, 1983, describe the use of those reconstituted particles for transferring a transforming polyoma DNA fragment to rat FIII cells. The empty capsids and reconstituted particles consist of all three of the polyoma capsid antigens VP1, VP2 and VP3 and there is no suggestion that pseudocapsids consisting of only the major capsid antigen VP1, could be used in genetic transfer.

Montross *et al.* 1991, described only the major capsid antigen, the cloning of the polyoma virus VP1 gene and its expression in insect cells. Self-assembly of empty pseudocapsids consisting of VP1 is disclosed, and pseudocapsids are said not to contain DNA. It is also reported that DNA inhibits the *in vitro* assembly of VP1 into empty pseudocapsids, which suggests that said pseudocapsids could not be used to package exogenous DNA for transfer to host cells. The results of Sandig *et al.* 1993, showed that empty capsids incorporating exogenous DNA could transfer DNA in a biologically functional manner to host cells only if the particles consisted of all three polyoma capsid antigens VP1, VP2 and VP3. Pseudocapsids consisting of VP1 were said to be unable to transfer the exogenous DNA so that it could be expressed in the host cells. Hagensee *et al* (1993) J. Virol., 67, 315-322 disclosed the production of L1 and L1/L2 human papillomavirus capsids, but did not suggest their use to carry exogenous material.

More recently, Forstova *et al* (1995) Human Gene Therapy, 6: 297-306 have reported the use of mouse polyoma pseudocapsids, consisting of the major capsid antigen VP1 without minor capsid antigens, to transfer heterologous DNA into rat cells, whereby the heterologous DNA was expressed.

Research pertaining to gene transfer into cells of the nervous system is one of the fastest growing fields in neuroscience. An important application of gene transfer is gene therapy, which is based on introducing therapeutic genes into cells of the nervous system by *ex vivo* or *in vivo* techniques. With the eventual development of efficient and safe vectors, therapeutic genes, under the control of a suitable promoter, can be targeted to the appropriate neurons or glial cells. Gene therapy is not only applicable to the treatment of genetic diseases of the nervous system and the control of malignant neoplasia, but it also has therapeutic potential for acquired degenerative encephalopathies (Alzheimer's disease, Parkinson's disease), as well as for promoting neuronal survival and regeneration in various pathological states.

The present invention aims to overcome the above problems associated with known viral and non-viral methods of exogenous material transfer by providing a method involving the use of a pseudocapsid consisting of a papovavirus major capsid antigen to transfer exogenous material to a host neuronal cell wherein that material is biologically functional.

The invention provides use of a pseudocapsid formed from a papovavirus major capsid antigen and excluding minor capsid antigens, which pseudocapsid has exogenous material associated therewith, in the manufacture of a pseudocapsid composition for treating a neuronal host cell so that the exogenous material is taken up by the cell and is biologically functional in that cell, provided that the pseudocapsid is not one formed

from at least one semi-purified or pure SV40 capsid protein and a constituent selected from the group consisting of an exogenous DNA encoding an exogenous protein or peptide product, or encoding a therapeutic RNA, or itself a therapeutic product, a vector comprising exogenous DNA encoding an exogenous protein or peptide product, or encoding a therapeutic RNA, or itself a therapeutic product, an exogenous RNA encoding an exogenous protein or peptide product or itself a therapeutic product, a vector comprising an exogenous RNA encoding an exogenous protein or peptide product, or itself a therapeutic product, an exogenous protein or peptide product, and antisense RNA, ribozyme RNA or any RNA or DNA which inhibits or prevents the expression of undesired protein/s in a mammalian cell, and optionally further comprising operatively linked regulatory elements sufficient for the expression and/or replication of said exogenous therapeutic RNA or of an exogenous protein or peptide in a mammalian cell.

Preferably neuronal host cell is derived from or present in the central nervous system of a mammal.

Preferably the neuronal host cell is derived from or present in the brain of a mammal.

Preferably the major capsid antigen is from one or more of human papilloma virus; mouse polyoma virus, bovine papilloma virus; simian virus 40; or the human polyoma virus BK.

Preferably the major capsid antigen is VP1 from mouse polyoma virus.

Preferably the biological functioning of the exogenous material in the host cell has a beneficial effect on the cell or a mammal containing that cell, especially the treatment and/or prevention of a neurological disease, such as Alzheimers' or Parkinsons' disease.

Preferably the exogenous material is genetic material and preferably comprises all or a coding part of at least one gene.

Preferably the gene is selected from one or more of nerve growth factor; tyrosine hydroxylase or neural apoptosis-inhibitory protein.

Preferably expression of the gene or genes is under the control of a nervous system-specific promoter or a promoter specific for a particular neuronal cell subpopulation.

Preferably the promoter is selected from the promoters for neurofilament light chain, neuron-specific enolase, tyrosine hydroxylase, dopamine β-hydroxylase, glial acidic-fibrillary protein, T$\alpha$1-tubulin, β-chain of platelet derived growth factor, myelin basic protein, and preproenkephalin.

This is useful because the biological function can be directed to the cell or tissue type of particular interest. Hence, side effects due to biological function in other cells or tissues are minimized.

In another aspect the invention provides a method of making a pseudocapsid composition comprising providing an empty pseudocapsid formed from papovavirus major capsid antigen and excluding minor capsid antigens; providing exogenous material; and mixing the empty pseudocapsid and exogenous material whereby the exogenous material becomes associated with the empty pseudocapsid; wherein the exogenous material is selected so that, in use, the biological functioning of the exogenous material in a neuronal host cell has a beneficial effect on the cell or a mammal containing that cell by treating and/or preventing a neurological disease, provided that the pseudocapsid is not one formed

from at least one semi-purified or pure SV40 capsid protein and a constituent selected from the group consisting of an exogenous DNA encoding an exogenous protein or peptide product, or encoding a therapeutic RNA, or itself a therapeutic product, a vector comprising exogenous DNA encoding an exogenous protein or peptide product, or encoding a therapeutic RNA, or itself a therapeutic product; an exogenous RNA encoding an exogenous protein or peptide product or itself a therapeutic product, a vector comprising an exogenous RNA encoding an exogenous protein or peptide product, or itself a therapeutic product, an exogenous protein or peptide product, and antisense RNA, ribozyme RNA or any RNA or DNA which inhibits or prevents the expression of undesired protein/s in a mammalian cell, and optionally further comprising operatively linked regulatory elements sufficient for the expression and/or replication of said exogenous therapeutic RNA or of an exogenous protein or peptide in a mammalian cell.

In another aspect, the invention provides a pharmaceutical composition comprising a pseudocapsid formed from a papovavirus major capsid antigen and excluding minor capsid antigens, which pseudocapsid has a pharmacologically active compound associated therewith; the pseudocapsid being provided together with a pharmacologically acceptable carrier, wherein the pharmacologically active compound is selected to have a beneficial effect on a neuronal host cell or a mammal containing the cell by treating and/or preventing a neurological disease, provided that the pseudocapsid is not one formed

from at least one semi-purified or pure SV40 capsid protein and a constituent selected from the group consisting of an exogenous DNA encoding an exogenous protein or peptide product, or encoding a therapeutic RNA, or itself a therapeutic product, a vector comprising exogenous DNA encoding an exogenous protein or peptide product, or encoding a therapeutic RNA, or itself a therapeutic product, an exogenous RNA encoding an exogenous protein or peptide product or itself a therapeutic product, a vector comprising an exogenous RNA encoding an exogenous protein or peptide product, or itself a therapeutic product, an exogenous protein or peptide product, and antisense RNA, ribozyme RNA or any RNA or DNA which inhibits or prevents the expression of undesired protein/s in a mammalian cell, and optionally further comprising operatively linked regulatory elements sufficient for the expression and/or replication of said exogenous therapeutic RNA or of an exogenous protein or peptide in a manunalian cell.

Preferably the pharmaceutical composition is adapted for intranasal or intraperitoneal or intravenous or subcutaneous administration.

By "pseudocapsid" we mean a structure in which the only papovavirus capsid antigen is the major capsid antigen without minor capsid antigens, which structure is hollow so that it can contain exogenous material.

The term "papovirus" defines a general family of viruses including simian virus 40 (SV40), polyoma virus (a mouse virus), human variants (such as BK and JC) and papillomaviruses including human and bovine variants and other members. In each case, there is a major capsid antigen and one or more minor capsid antigens. For example, in papillomavirus the major antigen is L1 and the minor antigen is L2. In the other paporaviruses the major capsid antigen is VP1. In the present invention, the "pseudocapsids" are formed from the major capsid antigen and not the minor antigen(s).

The term "major capsid antigen" includes conservative variants thereof capable of assembling into a pseudocapsid and preferably having an antigenic determinant in common with the wild-type antigen. The capsid antigen can be a hybrid of polyomavirus major capsid antigens, for example a hybrid of human BK virus major capsid antigen and mouse polyoma virus VP1.

By the exogenous material being "associated with" the pseudocapsid, we mean that the material is protected thereby. For example, exogenous DNA will be protected from degradation by DNases such as DNaseI, and exogenous protein will be protected from proteases. The exogenous material can be enclosed within an empty pseudocapsid or otherwise wrapped up with the capsid antigen.

The term "exogenous material" as used herein means material other than wild type papovirus nucleic acid. Preferably, the material is genetic material, for example DNA.

The term "neuronal cell" is intended to embrace cells of the nervous system of a mammal, particularly cells of the central nervous system (CNS) which lie within the blood-brain barrier (bbb), especially cells of the brain. Preferred neuronal cells include neurons from spinal cord, cerebellum, basal ganglia, thalamus, hippocampus, substantia nigra, neocortex, endothelial cells derived from the neural crest, foetal neurons, neuronal multipotent cell lines, adrenal chromaffin cells, striatum, glial cells, myoblasts or fibroblasts. Preferred neuronal cells are differentiated neurons which cannot divide.

The term "beneficial effect" is intended to embrace a therapeutic effect as well as other effects which may have utility for non-therapeutic applications. For example, biological functioning of the exogenous material may allow easy detection of a desired cell type. Such detection could be valuable for diagnostic applications as well as for experimental studies of neuronal cells.

The term "neurological disease" is intended to embrace any disease which is caused or effected by the physiological state of a cell of the nervous system of a mammal, especially a human. Examples of such neurological diseases includes Alzheimers', Parkinsons', Gliosis, Dysautonomia, and Tomaculous neuropathy.

The inventors have demonstrated that a simplified carrier consisting of a pseudocapsid involving only the major papovaviral capsid antigen can package and protect exogenous DNA, and transfer it into cells in a manner that allows for functional expression. A well-characterised gene, an efficiently transforming mutant form of the mouse polyoma virus middle T-antigen (MT), designated *dl8*MT (Griffin and Maddock, 1979), was chosen to show the worth of this system. The *dl8*-derived antigen possesses distinct physical and biological properties, which allow it to be easily assayed and distinguished from any wild type viral gene product that might have inadvertently contaminated recipient cells.

Papovaviruses have a broad host range and permissive cells include mammalian cells, such as rodent and human cells (Pontén, 1971).

The inventors have demonstrated that the pseudocapsids can successfully transfer exogenous genetic material in human cells.

Physical, non-viral gene transfer methods such as chemical and mechanical techniques and membrane fusion-mediated transfer *via* liposomes have been recently reviewed and assessed (Morgan and Anderson, 1993). The pseudocapsid (in the preferred embodiment involving genetic material) approach of the present invention resembles these procedures in that only the DNA intended for study, and no unwanted viral information, is introduced into cells. Moreover, the efficiency of functional gene transfer achieved by the method of the invention has been found to be between fifty and a hundred times greater than that achieved using conventional transfection methods.

The present data show that, in contrast to the calcium phosphate - or liposome-mediated route, long-term stable gene transfer and expression can be accomplished using pseudocapsids. Finally, it should be noted that whereas greater quantities of exogenous DNA are introduced into recipient rodent cells by the calcium phosphate technique, the latter route produces greater heterogeneity in the delivered DNA than observed with the pseudocapsid approach, and is no more effective in terms of protein expression. The pseudocapsid approach is also advantageous in that it can be manipulated in a manner not applicable to chemical and physical transfer routes, making it of considerable value in future work including studies applied to gene therapy. The ease with which pseudocapsids can be produced and their efficient delivery of exogenous material, including the delivery of DNA to the cell nucleus, makes the present papovirus pseudocapsid method of delivery especially attractive for use in therapy, especially gene therapy and antibody or drug targeting. A particular advantage of the pseudocapsid method of transfer is that it can deliver functional exogenous material to the nucleus of a cell.

Preferably, the exogenous material is derived from an eukaryotic source, in particular from a mammal such as a rodent or a human. By material "derived from" we include material actually derived from a eukaryotic source, or obtained using eukaryotic material (for example cDNA obtained using eukaryotic mRNA) or synthesised to correspond to eukaryotic material (for example a chemically synthesised or recombinant copy of a eukaryotic cDNA, or an antisense oligonucleotide to eukaryotic genetic material).

Alternatively or additionally, the exogenous material may be a protein or other polypeptide, or any pharmacologically active compound, especially one which acts in or on the nucleus of cells.

In a preferred embodiment the exogenous material comprises a complex of two or more substances such as biological macromolecules. For example, if the sequence of the site to which DNA is to be targeted is known, this can be incorporated into the exogenous DNA together with the gene of interest, to promote homologous recombination at that site, using either double-stranded or single-stranded DNAs 20-200 bases long for this purpose. To promote the recombination event, single stranded recombinase binding proteins, such as the *Escherichia coli* RecA (B.J. Rao and C.M. Radding, Proc. Natl. Acad. Sci. U.S.A. 91, 6161-6165, 1994) or its counterpart from *S. Cerevisiae,* Rad51, (A. Shinohara, H. Ogawa and T. Ogawa. Cell 69, 457-470, 1992) can be attached to DNA. Such a method can be used to greatly increase the efficiency and specificity of DNA integration into the host cell genome.

To demonstrate the effectiveness of the above method, for example, the *dl8*MT transforming gene of polyoma virus, to which a 20-200 long segment of mouse DNA has been ligated, with or without attached recombinase (or its active polypeptide site) can be incorporated into pseudocapsids and the ability of this carrier to produce dense foci, a marker of cellular transformation, monitored *in vitro.* For the mouse DNA sequence the results of D.M. Ding, M.D. Jones, A. Leigh-Brown and B.E. Griffin, EMBO J. 1, 461-466, 1982; C.H. Streuli, N.S. Krauzewicz and B.E. Griffin, J. Virol. 64, 3570-3580 (1990) can be used. The comparative numbers of dense foci obtained are a marker of efficiency of the system and the effect produced by adding, for example, a recombinase protein. Sequence data obtained from chromosomal DNA from foci confirms the efficiency and specificity of the targeting. The present invention thus provides a method for specific targeting of exogenous DNA into a host chromosome.

A particularly preferred exogenous material for association with pseudocapsids of the invention comprises peptide-nucleic-acids (PNAs).

Peptide nucleic acids (PNAs) are single chain compounds consisting of a polyamide backbone having purine or pyrimidine bases attached to side chains, to form a base sequence superficially similar to that of a single chain nucleic-acid. Their structure, composition and synthesis is described by Nielsen, *et al* (1991). Science, 254, 1497-5001; Egholm *et al* (1992). American Chem. Soc. 114; and Cherny *et al* (1993). Proc. Natl. Acad. Sci. USA, 90, 1667-70.

PNAs are preferred exogenous material because they have properties that make them useful in therapy of diseases or to modify the structure of the genome of living cells by affecting gene expression.

In living cells, by binding one strand of complementary DNA in a duplex, and thereby displacing a portion of the other strand to form a D loop structure, PNAs can inhibit transcription of a gene, or cause DNA breakage as a consequence of the cellular enzymatic machinery attempting to repair the damage. Such breakage is a focus for an increased level of recombination with any homologous DNA strands present in the same pseudocapsid or delivered separately in another pseudocapsid. Such breakage can also kill the organism to which the DNA belongs. The organism either is the cell itself, or a protozoan parasite or a virus, etc. Since it is possible to synthesise unique PNA moieties having sequence specificities specific for pathogens such as viruses, including those like the retroviruses which may integrate their DNA into the genome of the host, PNAs can be used to selectively attack the target pathogen without any deleterious effect on the host genome.

Particularly preferred proteins or polypeptides are antibodies, recombinant antibodies, or fragments thereof.

The variable heavy ($V_H$) and variable light ($V_L$) domains of the antibody are involved in antigen recognition, a fact recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison *et al* (1984) *Proc. Natl. Acad. Sci. USA* **81**, 6851-6855).

That antigenic specificity is conferred by variable domains and is independent of the constant domains is known from experiments involving the bacterial expression of antibody fragments, all containing one or more variable domains. These molecules include Fab-like molecules (Better *et* al (1988) *Science* **240**, 1041); Fv molecules (Skerra *et al* (1988) *Science* **240,** (1038); single-chain Fv (ScFv) molecules where the $V_H$ and $V_L$ partner domains are linked via a flexible oligopeptide (Bird *et al* (1988) *Science* **242,** 423; Huston *et al* (1988) *Proc. Natl. Acad, Sci,* USA **85,** 5879) and single domain antibodies (dabs) comprising isolated V domains (Ward *et al* (1989) *Nature* **341,** 544). A general review of the techniques involved in the synthesis of antibody fragments which retain their specific binding sites is to be found in Winter & Milstein (1991) *Nature* **349,** 293-299.

By "ScFv molecules" we mean molecules wherein the $V_H$ and $V_L$ partner domains are linked via a flexible oligopeptide.

The advantage of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')$_2$ fragments are "bivalent". By "bivalent" we mean that the said antibodies and F(ab')$_2$ fragments have two antigen combining sites. In contrast, Fab, Fv, ScFv and dAb fragments are monovalent, having only one antigen combining site.

Preferably, the pharmacologically active compound is a chemotherapeutic agent.

Chemotherapeutic agents are used to eliminate cancerous tissue. Recently, agents in the form of proteins and other biological products including molecules of the immune system such as tumour necrosis factor, interleukins, interferons and monoclonal antibodies have been used for chemotherapy.

As discussed by Jain, R.K. (Scientific American July 1994: 42-49), to eradicate tumours chemotherapeutic agents must disperse throughout the growths in sufficiently high concentrations. However, it has been found that tumours are resistant to such dispersal. Conventional chemotherapeutic agents are small (molecular weight lower than 2000 daltons) and when administered to a mammal, leave the blood vessels and migrate through normal tissue mainly by diffusion. However, the above biological products are larger molecules (molecular weight above 5000 daltons) and travel mainly by convection, that is, transport in a stream of flowing fluid.

Convection - dependent agents are inhibited from dispersing through the tumour because the uniform pressure in the tumour's interior prevents convection from operating. This may explain why such agents display disappointing tumour eradication results *in vivo.*

Both large and small chemotherapeutic agents are susceptible to degradation before they reach their target cancer cells. Repeated dosage is therefore required to maintain the sufficiently high concentrations needed for tumour eradication. This is expensive and may produce an immune response that degrades the agent before it has the chance to work.

Other tumour barriers which reduce the effectiveness of chemotherapeutic agents include the lack of oxygen in tumours which may cause them to secrete large amounts of lactic acid. Many agents are broken down or fail to work in an acidic environment.

A current strategy for overcoming the above problem is to link an enzyme with an antibody specific for a tumour cell antigen to form an "abzyme". The enzyme is capable of converting a prodrug into an active form capable of killing a tumour cell. Large amounts of the abzyme are injected in the blood stream so that it can accumulate in the tumour. The prodrug is injected once the abzyme has cleared from normal tissues. As the prodrug is small it can diffuse into the tumour and be activated by the abzyme to kill cancer cells.

However, this strategy is not wholly effective because the prodrug can be degraded quickly and can seep back into vessels and be cleared away from tumours just as easily as it can diffuse out of blood vessels.

The pseudocapsid approach for delivering chemotherapeutic agents to neuronal cells is advantageous because it protects the agents from degradation, during transport to target cancer cells.

Preferably, the exogenous material comprises all or a coding part of at least one gene. Advantageously, expression of the gene in the host cell has a therapeutic effect on the cell and/or a multi-cellular organism comprising that cell by treating and/or preventing a neurological disease.

Preferred genes for transfer to neuronal cells according to the invention include tyrosine hydroxylase, preferably human tyrosine hydroxylase; the neural apoptosis-inhibitory protein gene (Roy *et al*, 1995, cell 80:167); and the nerve growth factor gene.

If the exogenous material is protein it is protected from degradation by a protease. If the exogenous material is a pharmacologically active compound, it is protected from degradation caused by the surrounding environment.

The protection of exogenous material by the pseudocapsid from degradation can be determined readily. For example, to establish the levels of protection for DNA, the pseudocapsids can be packed with a radioactively labelled fragment, the buffer adjusted to 10mM MgCl$_2$ and then exposed for 20 min at 37°C to DNase I (100/µg/ml). The DNase treated sample is then fractionated on a 10-40 % sucrose gradient made in buffer A (see below) lacking glycerol to separate protected from digested DNA. The refractive index and radioactivity of collected fractions is measured. Most radioactivity is unincorporated DNA and is found on the top of gradient. Radioactive peaks banding within gradients corresponding to pseudocapsids (as shown by electron microscopy) indicate that the labelled DNA and pseudocapsids are associated and affording protection to the DNA. The amount of protected DNA typically represents 2-5 % of total DNA measured.

Exogenous material may be packaged, that is, incorporated into an empty pseudocapsid. By "empty" we mean that the pseudocapsid does not contain any papovavirus genetic material. It may of course contain other material, such as protein.

The attachment of protected exogenous material to the pseudocapsid, for example by coating with charged molecules, is a preferred method of associating the material with the empty pseudocapsid because it enables larger amounts of material to be transferred.

For example, larger fragments of exogenous genetic material can be packaged using an *in vitro* method such as that described below, and then further protected by the addition of charged molecules (such as poly-L-lysine or basic histones), followed by incubation for 30 mins at 37°C.

Binding charged molecules causes exogenous genetic material such as DNA to alter its conformation which affects the amount of genetic material that can be packaged into empty pseudocapsids.

The pseudocapsids used may be provided in the form of an icosahedron, like the wild type papovavirus capsid, or larger spherical or filamentous shapes which are advantageous because they permit packaging of larger nucleic acid fragments. The term "pseudocapsid" is thus not limited to particles which closely resemble wild type papovavirus capsids.

The transfer of larger amounts of exogenous material may be desirable. For example transfer of larger fragments of genetic material is advantageous because there appears to be a size constraint on the amount of genetic material that can be packaged and transferred to host cells, using the empty icosahedral VP1 pseudocapsids, for example. The wild type viral genome of papovaviruses is 5.2kbp and, using the passive DNA packaging procedures of Barr *et al.* (1979) or Slilaty *et al* (1982), the amount of DNA that can be packaged is around 3kbp. Whereas this size would be adequate for expression of many mammalian cDNAs, or, for example, oligonucleotides to be used for antisense "knockout" experiments (ie where a gene function is deleted), it would preclude packaging of a number of mammalian genes in their entirety.

Recombinant baculoviruses carrying genes for papovaviruses major capsid antigen, such as the mouse polyoma virus major capsid antigen VP1, have been constructed and the antigens can be expressed in insect cells as described by Forstová *et al.* 1993. In this method insect cells are infected with a recombinant baculovirus expressing VP1, harvested 72 hours post infection, resuspended in buffer A (150mM NaCl, 10mM Tris-HCl, pH 7.4, 0.01mM $CaCl_2$, 0.01 % Triton X-100, 5% glycerol) and disrupted by sonication. Cell debris is removed by low speed centrifugation and VP1 pseudocapsids purified on a CsCl density gradient. Peak fractions of pseudocapsids are subjected to centrifugation through a sucrose gradient (10-40%) and peak fractions (monitored by immunoblotting and electron microscopy) are then concentrated by CsCl gradient centrifugation. After each centrifugation the pseudocapsids are dialysed into buffer A.

In a preferred method, Sf9 insect cells are incubated at a multiplicity of infection of 10-20 with a recombinant baculovirus expressing VP1, harvested 72 hrs postinfection, resuspended in buffer A (150mM NaCl, 10 micromolar $CaCl_2$, 10mM Tris-HCl, pH 7.6, 5% glycerol, 0.01%, Triton X-100) and disrupted by sonication. Cell debris is removed by low speed centrifugation at 8000 xg. The empty VP1 pseudocapsids in the supernatant are concentrated by pelleting through a sucrose cushion and then by banding on a CsCl gradient. The fractions containing purified empty pseudocapsids, as revealed by refraction index measurements or electron microscopy, concentrated through a second sucrose cushion.

Using the above methods the yield of empty VP1 pseudocapsids is at least ten times greater than that achieved when the same procedure is carried out by co-infection of insect cells with recombinant baculoviruses expressing VP1, and the minor capsid antigens VP2 and VP3. Furthermore, empty VP1 pseudocapsids are just as efficient as VP1, VP2 and VP3 capsids at packaging exogenous genetic material.

Empty pseudocapsids formed from other papovavirus major capsid antigens can be produced using similar methods.

Protecting the exogenous material with an empty pseudocapsid is conveniently achieved for exogenous DNA, for example, by mixing 1μg DNA with 10-60μg of empty pseudocapsids and incubating the mixture in 150mM NaCl, 0.01mM $CaCl_2$ 10mM Tris, at pH 7.4, 0.01 % TX-100, for 10 minutes at 37°C. The mixture is then subjected to osmotic shock by quick dilution with water to a final concentration of 35mM NaCl, with incubation for an additional 20 minutes. The pseudocapsids incorporating exogenous DNA can then be used in the exogenous material transfer method of the invention.

Exogenous material, in the form of protein and polypeptides such as antibodies and fragments thereof, and pharmacologically active compounds, can be packaged inside an empty pseudocapsid by a similar procedure in which the DNA is substituted in the above method by the exogenous material to be packaged.

Alternatively, exogenous material is associated with empty pseudocapsids by dissociating the empty pseudocapsid structure and reassembling it in the presence of the material.

An advantage of the pseudocapsids of the invention is that they are easier to produce than capsids containing both major and minor capsid antigens. This is because it is difficult to control the proportions of the various capsid antigens in the assembled capsid. Consequently, the properties of the capsids produced will vary and this unpredictability is undesirable.

A further advantage of the present pseudocapsids for *in vivo* exogenous material transfer over known capsids is that they are less likely to evoke a strong immune response than capsids because they only contain a single foreign antigen, the major capsid antigen.

Treatment of host cells with pseudocapsids in accordance with the invention can be carried out by washing the cells in serum-free medium and incubating them with pseudocapsids diluted to 1ml/$10^6$ cells with tissue culture medium. Following 90 mins incubation, medium containing serum is added to the culture. For example, 15μg of pseudocapsids containing an agarose gel-purified fragment (2.5μg) of the cellular gene p43 (Koch *et al.,* 1990) under the control of the cytomegalovirus immediate early promoter can be used to treat human embryo lung (HEL) fibroblast cells, using 2x$10^5$ cells. The cells are then incubated at 37°C for 72 hours post treatment.

To test for functioning of the p43 fragment, cells are harvested by washing with phosphate buffered saline (PBS), and lysing in Laemmli sample buffer (Laemmli, 1970). Proteins are fractionated on 10% SDS-PAGE, immunoblotted and probed initially with a p43 specific monoclonal antibody, then anti-β tubulin antibody (Sigma). Immune complexes are detected with an HRP conjugated secondary antibody (Dako plc) and chemiluminescent ECL reagent (Amersham plc).

## DESCRIPTION OF THE DRAWINGS

### Figure 1 (A)

Schematic presentation of the polyoma virus DNA fragment incorporated into pseudocapsids. The linear *Bcl*I-*Eco*RI (1680 b.p.) DNA fragment from pPyMT*dl8*, containing the polyoma virus origin of replication (ori), promoter (p) and the gene for the middle T antigen *dl8* deletion mutant (as noted below), was gel purified before use in DNA packaging and transfection experiments. Polyoma virus sequence numberings are those of Soeda *et al.* (1980). Cleavage sites for restriction enzymes are noted.

### Figure 1 (B)

Electron microscopy of self-assembled icosahedral VP1-pseudocapsids isolated from insect cells. Bar = 50 nm.

### Figure 1 (C)

Protection of packaged DNA by pseudocapsids from DNaseI cleavage.

The tracks contain: 1 and 2, control reactions with 1μg DNA, in the absence of pseudocapsids, without and with DNaseI treatment, respectively; 3 and 4, packaging mixture with 1μg DNA, without and with DNaseI treatment, respectively.

### Figure 2

Morphology and soft agar assay of cell lines cloned from foci of transformed cells, phase contrast microscopy of:

### Figure 2 (a)

the parental untreated rat-2 cell line;

### Figure 2 (b)

an example of a transformed cell line obtained from a focus formed following calcium-phosphate transfection; or

### Figure 2 (c)

cells isolated from a dense focus derived from pseudocapsids packaged with *dl8*MT DNA.

### Figure 2 (d)

shows a transformed colony from a pseudocapsid derived cell line growth in soft agar.

### Figure 3

Analysis of expression of the *dl8* middle T antigen (D18MT) gene in transformed cell lines.

### Figure 3(A)

Immunoblot of cell lysates probed with PAb 701, a monoclonal antibody specific for the N-terminal region of the three polyoma virus early antigens.

### Figure 3 (B)

*In vitro* kinase reaction of immunoprecipitates of cell lysates using Pab701, which does not recognise MT complexed to *c-src* (C-tracks) or Pab721, a monoclonal antibody which precipitates protein kinase active complexes of MT and *c-src* (M-tracks).

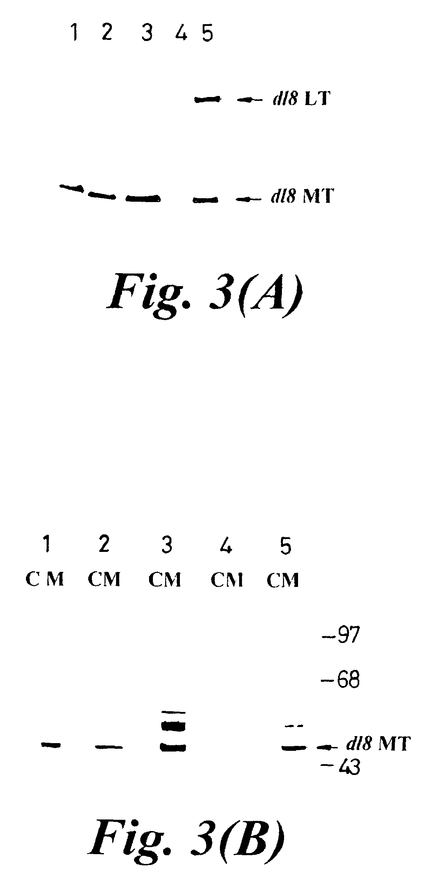

In Figures 3(A) and 3(B), the gels contain protein from: (tracks 1), the cloned cell line, C1, derived from calcium phosphate transfection; (tracks 2 and 3), cell lines P1 and P2 from pseudocapsid treated cells; (tracks 4), the parental rat-2 line; and, (tracks 5), rat-2 cells transformed with mutant *dl8* polyoma virions. The location of the *dl8*MT (and LT, where observed) is noted, as are molecular size markers (at right).

**Figure 4**

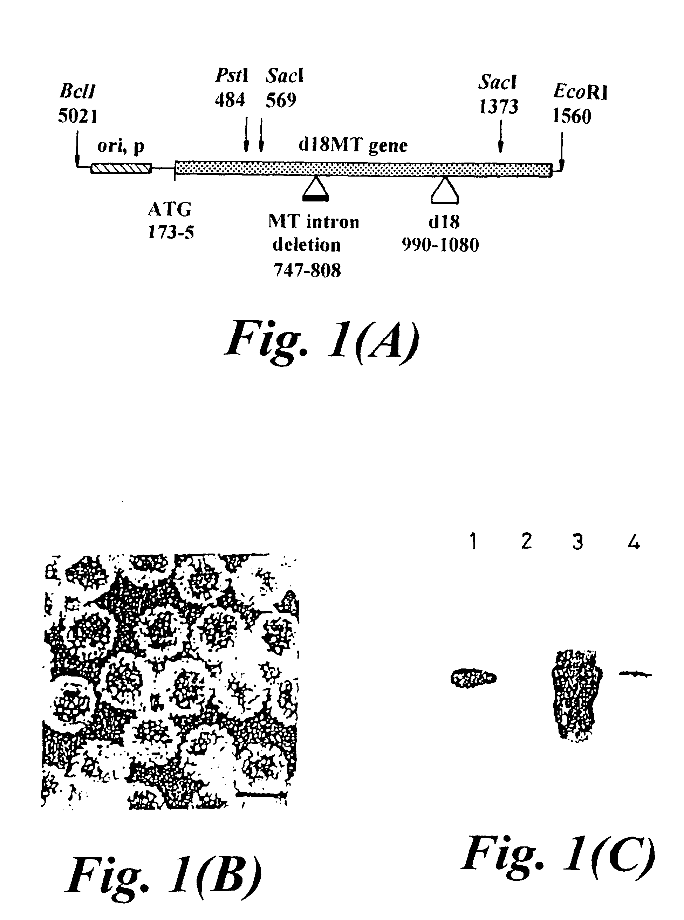

Southern blot analysis of transformed cell lines. Tracks contain: (a, e and i), DNA from cell line C1 (see above), generated by calcium phosphate precipitation, cleaved with *Hin*dIII (no site in viral DNA), with *Pst*I (one site) and with *Sac*I (two sites), respectively; (tracks b, f and j), and (c, g and k), DNA from two of the individual transformed foci generated with polyoma pseudocapsids, cleaved respectively with *Hin*dIII, *Pst*I and *Sac*I; tracks (d, h and 1), DNA from control rat-2 cells cleaved with the same enzymes; track m, the *Bcl*I-*Eco*RI input fragment (1ng). Higher molecular weight bands probably reflect integrated viral DNA. The location of the 654 bp internal *Sac*I fragment of the MT gene indicative of the efficiency of exogenous DNA, (figure 1A) is noted (●). Migration positions of molecular weight markers (in kb) are indicated.

**Figure 5**

Western blot analysis of the enhanced expression of p43 after pseudocapsid gene transfer to HEL cells.

**Figure 5 (A)**

The tracks contain cells treated with: (1 and 4), the p43 DNA-carrying pseudocapsids; (track 2), empty pseudocapsids; (track 3), DNA only; or, (tracks 5 and 6), DNA co-precipitated with calcium phosphate. Tracks 2 and 3 probably reflect the levels of endogenous p43.

**Figure 5 (B)**

The same gel probed with an anti-tubulin antibody to indicate loading of total protein in the individual tracks.

**Figure 6**

A schematic map of the plasmid pPyMT*dl8* containing the genome of the polyoma *dl8* mutant virus.

**Figure 7** Electron microscopy of self assembly pseudocapsids.

Icosahedral and tubular pseudocapsid assemblies were isolated from insect cell nuclei by differential centrifugation and observed by negative staining electromicroscopy.
Bar = 100 nm.

**Figure 8** DNA associated with pseudocapsids in the presence and absence of poly-L-lysine.

7.2 kbp supercoiled pCMVβ DNA, containing an expression cassette for the β-galactosidase gene, was incubated for 10 minutes alone (panel A), with pseudocapsids at a ratio of 1:30 (w/w) (panel B), pseudocapsids, followed by poly-L-lysine (panel C), or full pseudocapsids (panel D). Samples were spread in the presence of cytochrome C, transferred to copper grids, stained with uranyl acetate and observed by electron microscopy.

(Magnification = x 70,000).

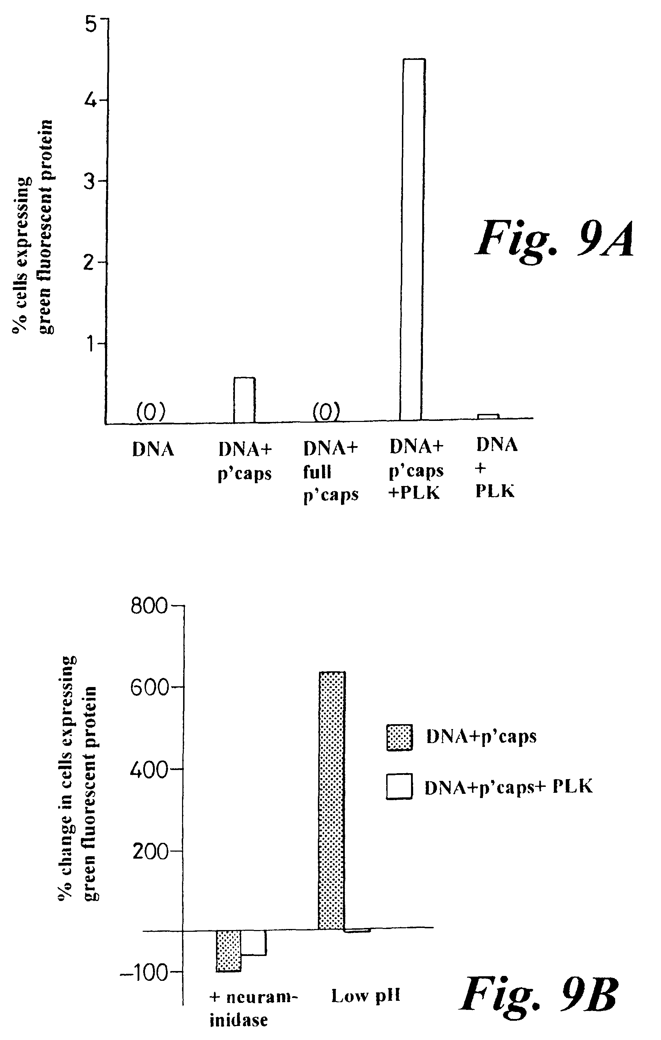

**Figure 9** Expression of marker gene encoding green fluorescent protein in Cos 7 cells *in vitro.*

A. Cos 7 monkey cells were cultured in the presence of DNA alone (DNA) DNA-pseudocapsid complexes (DNA+p'caps). DNA-full pseudocapsid mixtures (DNA+full p'caps). DNA-pseudocapsid-poly-L-lysine complexes (DNA+p'caps+PLK), or DNA-poly-L-lysine complexes (DNA+PLK), for 36 hours and cells expressing green fluorescent protein counted in each sample. Positive cells are expressed as a percentage of total cells per experiment. (O) denotes no positive cells counted per sample.

B. A comparison was made between expression of marker plasmid from DNA-pseudocapsid complexes (closed bars), or DNA-pseudocapsid-poly-L-lysine complexes (open bars), in the presence of reagents that influence genuine polyoma virion infectivity. The effects of neuraminidase (+neuraminidase), or incubation at pH 6 (Low pH) on pseudocapsid gene transfer are expressed as the percentage change in number of cells expressing green fluorescent protein under these conditions, compared to incubation under control conditions (ie. at pH 7.5 and in the absence of neuraminidase).

**Table 1A** Expression of the marker gene, β-galactosidase, in organs of athymic (nu/nu)mice injected subcutaneously with pCMVβ DNA, pseudocapsids, or pCMVβ DNA-pseudocapsid complexes at 1 and 6 weeks.

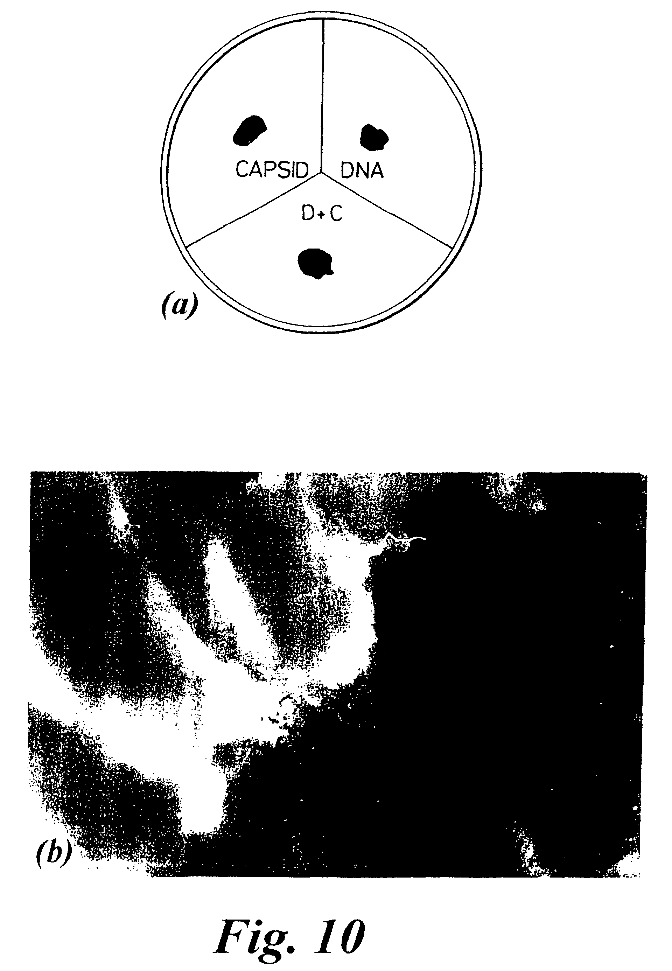

**Figure 10** Stable transfer of marker gene, β-galactosidase, to the central nervous system of the mouse.
**Panel A:** Athymic nu/nu mice were injected subcutaneously at the back of the neck with 150 μg of pseudocapsids (CAPSID), or 5 μg pCMVβ DNA (DNA), or pCMVβ DNA-pseudocapsid complexes (D+C). 6 weeks post injection mice were killed and the brain fixed and stained for β-galactosidase activity using X-gal as substrate.
**Panel B:** Sections of tissue from the brain of a mouse injected with DNA-pseudocapsid complexes were observed by light microscopy.

(Magnification: Panel A x 3/4: Panel B x 40)

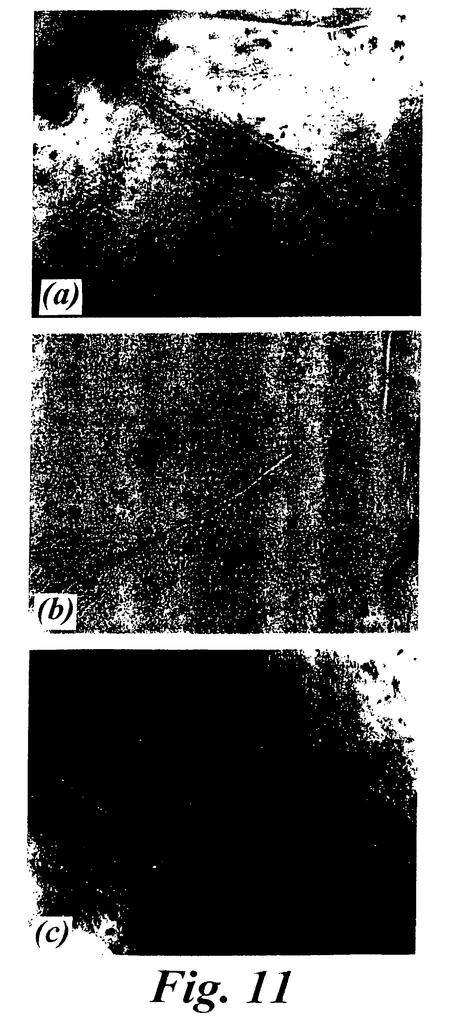

**Figure 11** Long-term expression of the marker gene, β-galactosidase, in rabbit corneal organ cultures.
Corneas were dissected from rabbits (female New Zealand White), quartered and incubated with pCMVβ DNA-pseudocapsid complexes (1 μg DNA: 30 μg pseudocapsids) (Panel A), 1 μg pCMVβ DNA alone (Panel B), or 1.5 x $10^7$ pfu/ml adenovirus recombinant expressing the β-galactosidase gene (Panel C). Corneas were incubated in tissue culture for 4 weeks and then fixed and stained for β-galactosidase activity using X-gal as a substrate.

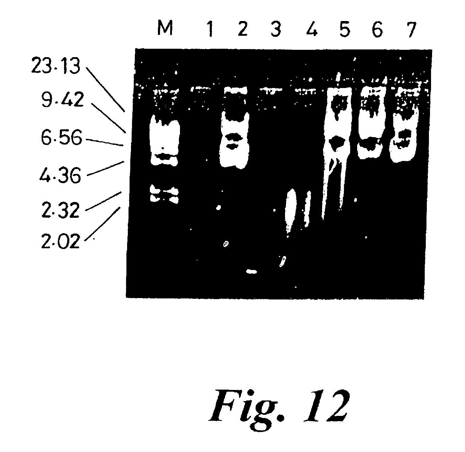

**Figure 12** Protection of exogenous DNA from DNaseI activity by pseudocapsids and polylysine. The gel contains: lane 1, untreated supercoiled 7.2kb pCMVβ DNA and, lanes 2 to 4, the same DNA treated with DNaseI after complexing with purified pseudocapsids (lane 2), or after initial treatment first with polylysine, then pseudocapsids (lane 3), or comlexed with polylysine subsequent to incubation with pseudocapsids (lane 4) . respectively, incubated at 37°C with $Mg^{2+}$ and $Ca^{2+}$. In addition to some apparent loss of DNA following DNAseI digestion, the predominant supercoiled form of the DNA (lane 1) is in part converted to circular and linear forms (lanes 3 and 4) during the course of the experiment. lane M contains molecular weight size markers. In similar experiments, using a mixture of 6.2 and 2.6 kbp DNA fragments, obtained by cleavage of pPyMT1 with *Eco*R1, data obtained (not shown) were consistent with the above results, with no preferred protection observed for the smaller of the two fragments.

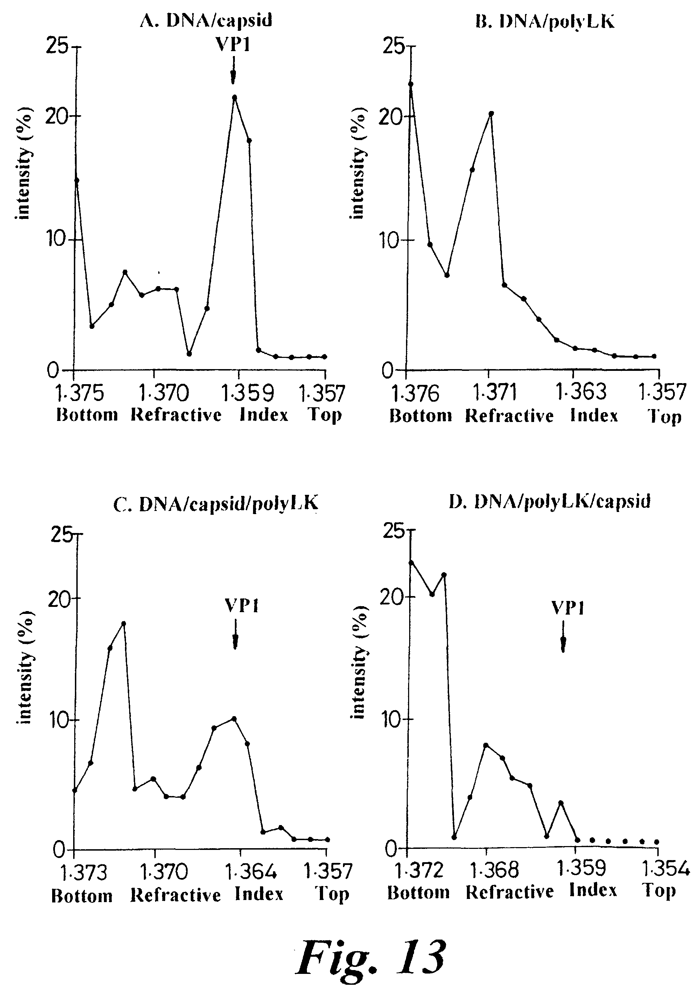

**Figure 13** CsCl density gradient profiles of DNA in complexes with pseudocapsids and/or polylysine. The pCMVβ DNA (7.2 kbp) treated with the designated reagents, was sedimented through a CsCl gradient (see Materials and Methods). Fractions containing DNA were detected by hybridization with $^{32}$P-labelled linearised pCMVβ DNA. Uncomplexed DNA sedimented at the bottom of the gradient. Signals were analysed by densitometry. A. DNA bound to pseudocapsids (DNA/capsid); B. DNA complexed with polylysine only (DNA/polyLK); C. DNA complexed first with pseudocapsids, followed by mixing with polylysine (DNA/capsid/polyLK); D. DNA complexed first with polylysine, followed by addition of pseudocapsids (DNA/polyLK/capsid). The fraction containing the majority of the pseudocapsids, noted by arrow,s was identified by its refractive index; the presence of VP1 was confirmed by Western blotting.

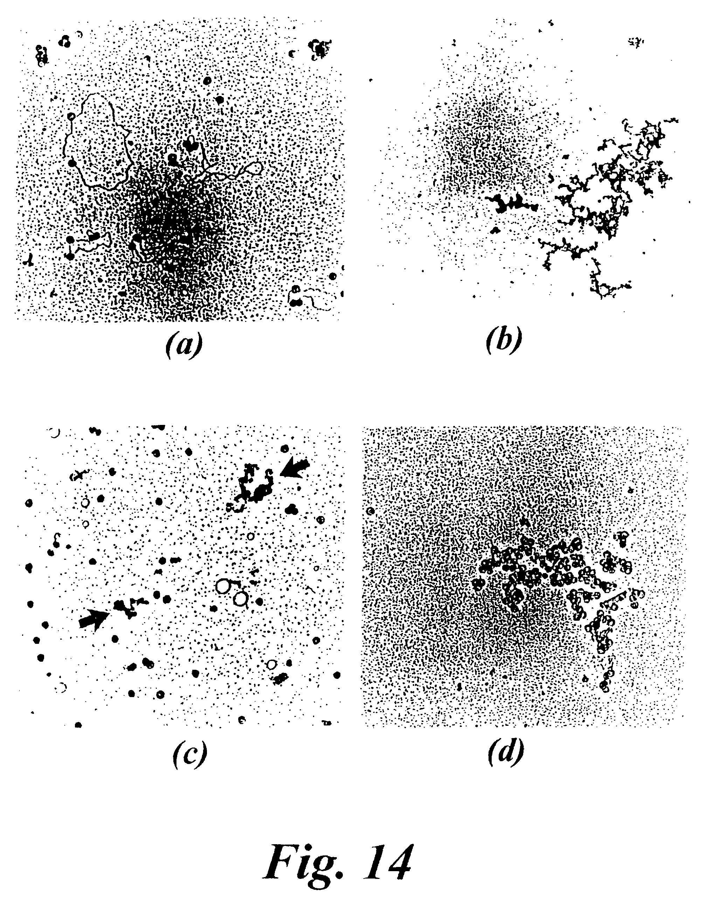

**Figure 14** Electronmicroscopy of complexes formed with DNA, pseudocapsids and polylysine. Complexes consist of: A. DNA and pseudocapsids obtained following osmotic shock; B. DNA and polylysine only; c. DNA and pseudocapsids mixed, then treated with polylysine; D. DNA and polylysine mixed, then treated with pseudocapsids. The arrow indicates ternary complexes in C.

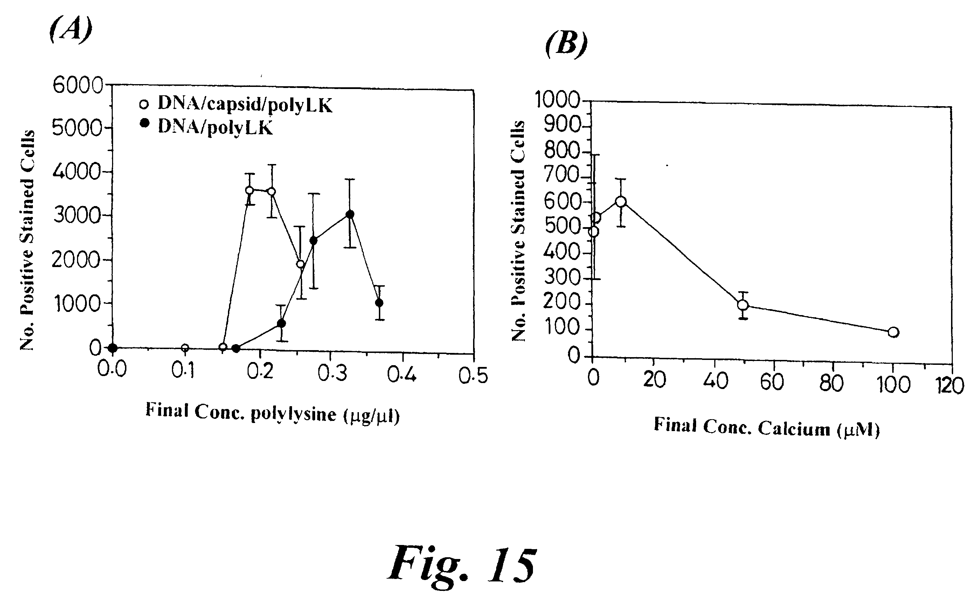

**Figure 15** Influences of polylysine (polyLK) or calcium ion concentrations on transfer efficiences. A. Complexes consisting of pCMVβ DNA and pseudocapsids were incubated at the indicated concentrations of polylysine, then exposed to osmotic shock; or B. the calcium concentration in the purification buffer was altered using VP1 pseudocapsids that were purified and stored in buffers with various concentrations of calcium (0, 5, 10, 50 or 100 μM). CCL 13 cells grown in 24 wells dishes were treated with the corresponding mixtures at indicated concentrations, and cells expressing the β-galactosidase gene were identified by the hisotochemical assay after 48 hours incubation at 37°C. The number of blue coloured cells were counted microscopically after overnight incubation in X-gal solution.

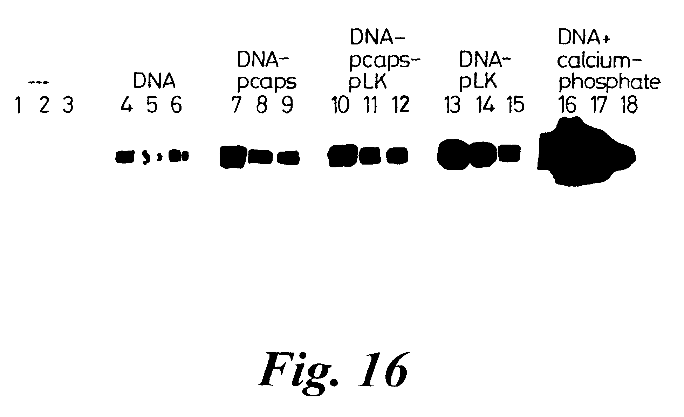

**Figure 16** Comparative β-galactosidase gene expression in rat 2 cells, as determined by RT-PCR. Cultures of rat 2 cells were treated with "naked" pCMVβ DNA or DNA/polylysine(pLK), pseudofected with pseudocapsids (pcaps), with or without polylysine, or transfected (using calcium phosphate). Isolated total RNA was used for synthesis of cDNA, which was amplified by PCR with oligonucleotide primers specific for the bacterial β-galactosidase gene. PCR products were separated by gel electrophoresis. The Southern blot was hybridized with a $^{32}$P-radiolabelled DNA probe and autoradiographed. lanes 1-3 contain cDNA from RNA extracted from untreated rat 2 cells - this represents cross-hybridizing endogenous levels of the transcript for β-galactosidase, at 2, 14 and 21 days, respectively. Lanes 4-18; data from separate experiments, as noted, each taken at three time points corresponding to 2, 14 and 21 days, respectively. Lanes 4-6, cells treated with DNA only; lanes 7-9, cells pseudofected with DNA/ pseudocapsid complexes, or, lanes 10-12 with the same complexes in the presence of polylysine; lanes 13-15, cells treated with DNA complexed only with polylysine, or (lanes 16-18) transfected with DNA using the calcium phosphate precipitation protocol.

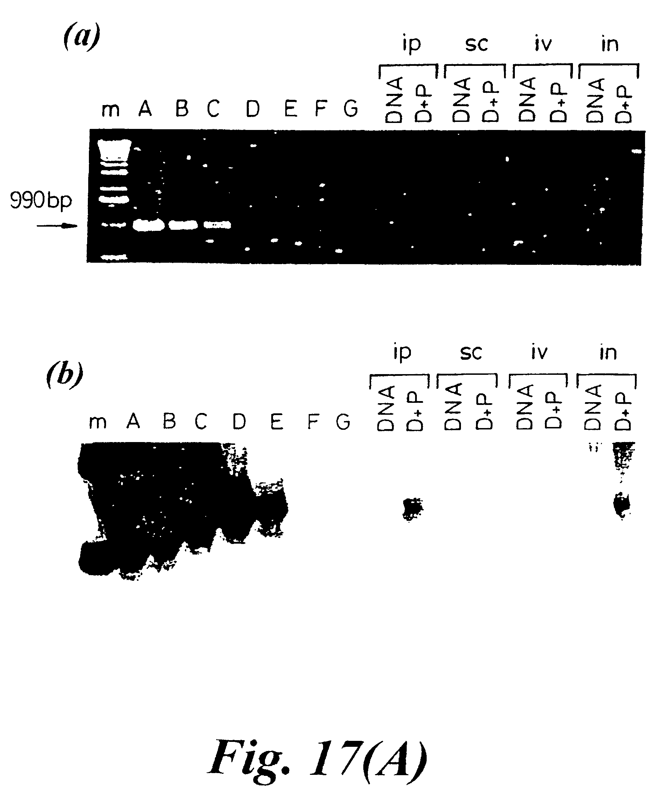

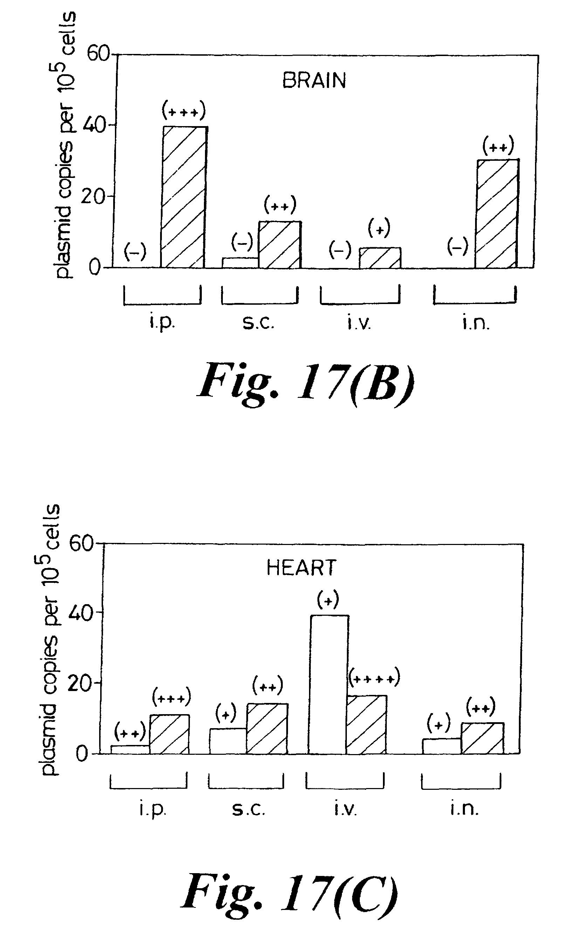

**Figure 17** Effect of the route of administration on *in vivo* gene transfer of β-galactosidase to heart and brain.

**A**. Nude mice treated intraperitoneally (i.p.), subcutaneously (s.c.), intravenously (i.v.), or intranasally (i.n.), with 5μg pCMVβ DNA alone, or with pseudocapsid-DNA complexes (150μg/5μg), and killed 7 weeks later. Panels: (a), ethidium bromide staining of PCR products separated by gel electrophoresis; (b), autoradiograph of Southern blot of a gel hybridised with radiolabelled pCMVβ. Tracks A-F: PCR products from $10^6$, $10^5$, $10^4$, $10^3$, $10^2$ and o copies pCMVβ, respectively, mixed with 500ng DNA from untreated mouse brain (990 bp, marked by arrow), track G: no template, track m: molecular weight markers. PCR products from 500ng brain DNA from mice treated with either pseudocapsid-DNA complexes (D+P), or DNA alone (DNA), as indicated.

**B and C**. Brain (panel B) and heart (panel C), dissected from mice treated as above, and tested for expression of β-galactosidase by X-gal staining, where (-) indicates no stained areas of tissue and (+), (++), (+ + +), (+ + + +) indicate increasing numbers of positively stained areas, as identified by light microscopy and β-galactosidase specific primers (DNA was amplified from a quarter of the tissue using pCMV specific primers). Black bars: pseudocapsid-DNA treated mouse tissue PCR products; white bars; DNA alone treated mouse PCR products.

**Table 2A** The influence of administration route on expression of marker gene *in vivo.*

Preferred embodiments of the invention will now be described, by way of example only, with reference to the above figures.

**Example 1: *Preparation of VL-YP1 recombinant baculoviruses and isolation of polyoma virus VP1 empty pseudocapsid particles.***

*Escherichia coli,* strain DH5, was used for the propagation of transfer vectors and recombinant plasmids. Where a digestion with the *BclI* enzyme was used, plasmids were propagated in *E. coli* JM110 strain (a *dam dcm* mutant derivative of JM 101) (Yanish-Perron et al., 1985, Gene **33**: 103-119).

The baculovirus transfer vectors p VL 941 containing a *Bam* HI cloning site, and p VL 1393 containing a multiple cloning site polylinker were used for the construction of recombinant plasmids where polyoma VP1 was placed under control of the polyhedrin promoter. Plasmid pMJA2 containing the whole polyoma virus genome inserted into the *Eco*RI site (Krauzewicz *et al.,* 1990) was used for isolation of the gene.

The recombinant plasmid pVL-VP1 was constructed by ligation of the *Eco*RV - Xbal 1584 b.p. fragment of the pMJA2 plasmid (polyoma virus numberings 4106 *Eco*RV to 2522-Xbal are those of Soeda *et al.,* (1980) with vector pVL1393, cleaved with *Sma*I and *Xba*I. Competent bacteria, DH5, were transformed with ligation mixture and plasmid pVL-VP1 was selected.

*Spodoptera frugiperda* (Sf9) cells were grown as monolayer cultures at 27°C in standard TNM-FH medium containing 10% foetal calf-serum (FCS), as described by Hink (1970, Nature: 226: 466-467). *Autographa californica* nuclear polyhedrosis virus (AcNPV) was propagated as described by Summers (1987, Texas agricultural Bulletin No. 1555, Texas agricultural Experiment Station). A recombinant baculovirus containing polyoma VP1, was prepared by *in vivo* allelic exchange between wild type AcNPV DNA and the individual DNA of recombinant transfer plasmid pVL-VP1 , according to the method described previously (Forstova *et al.,* 1989). Sf9 cells were infected with 10 plaque forming units per cell of recombinant baculovirus. A monolayer of cells was washed with TNM-FH medium and the inoculum of virus was added. Cells were rocked at rom temperature for 1 hr. Complete medium with 10% FCS was then added and cells were incubated at 27°C.

*Isolation of empty polyoma virus pseudocapsids from insect cells.* Insect cells were infected with VL-VP1 baculovirus. Cells were harvested 72 p.i., resuspended in buffer A (10 mM Tris-HCl [pH 7.6], 150mM NaCl, 10 micro-molar $CaCl_2$ 5% glycerol, 0.01 % Triton X-100), and disrupted by sonication (10 amplitude microns [MSE Soniprep 150] for 3 secs). Cell debris was removed by centrifugation (20 min. 8,000 x g) and particles purified by centrifugation though a sucrose cushion (20 % , made in buffer A lacking glycerol), centrifuged at 150000 xg for 3 hours. The pellet was resuspended in buffer A, lacking glycerol and sonicated briefly.

CsCl was added to a density of 1.30-1.33 g/ml and the gradient formed by centrifugation for 20 hours at 150000 x g. Peak fractions, of densities between 1.30 and 1.35 g/ml were collected and concentrated through a second sucrose cushion.

*Electron microscopy* Formvar (EMSCOPE)-coated grids were incubated for several seconds with 10-µl pseudocapsid samples, drained of surplus liquid, transferred to a drop of 3 % phosphotungstic acid (pH 6.6), and left for 45 s. The grids were dried and stored at room temperature.

VP1 pseudocapsids from other papovaviruses can be prepared using the above protocol except that the VP1 gene of the papovavirus of interest replaces the polyoma VP1 gene in the baculoviral expression vector. The VP1 gene sequences of other papovaviruses are disclosed in the following references:

| PAPOVAVIRUS | REFERENCE |
|---|---|
| BK (dunlop strain) | Cell 18; 963-977 (1979) (revisions Cell 19; 567-567, 1980) |
| SV40 | Science 200; 494-502 (1978); Nature, 273; 113-120(1978) |
| bovine (Bo) | J Gen Virol 71; 1723-1735 (1990) |
| Lymphotrophic (Lym) | Virology 143; 196-211 (1985) |
| Mouse: A2 A3 CSP Kilham | Nature 283; 445-453 (1980) Cell 17; 715-724 (1979) J Virol 48; 472-480 (1983) Virology 181; 469-480 (1991) |

**Example 2: *Packaging of polyoma virus dl8 MT gene into VP1 pseudocapsids.***

Cells: The *dl8* virion transformed line, as control, was isolated from foci formed following infection of rat-2 cells with the *dl8* mutant virus. Both these cells and the parental rat-2 cell line were passaged in complete Dulbecco's modified Eagle's medium (DMEM) containing 10% foetal calf serum.

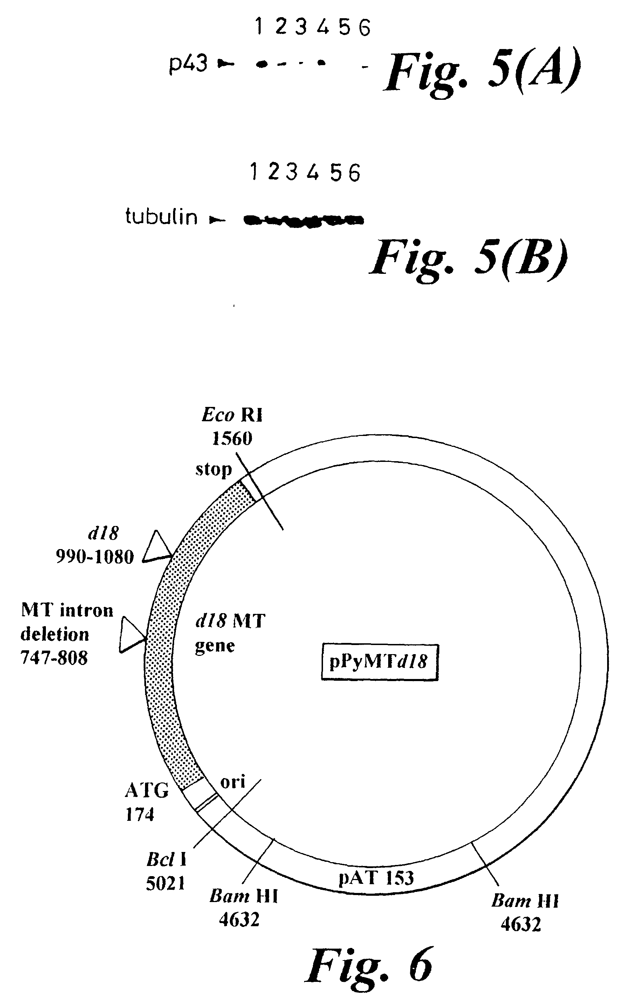

The intron of the gene for polyoma virus middle T antigen was deleted by site directed mutagenesis (see Figure 6 and Strauss *et al.,* 1986). A *Bcl*I/*Eco*RI linear DNA fragment was packaged into pseudocapsids according to the procedure of Barr *et al.* (1979): briefly, 1µg agarose gel-purified DNA was mixed with 10-60 µg of empty pseudocapsids and incubated in 150 mM NaCl, 10 micro M $CaCl_2$, 10 mM Tris, pH 7.6, 5% glycerol, 0.01 % Triton X-100 for 10 min. at 37°C. The mixture was then exposed to osmotic shock by quick dilution with water to a final concentration of 35 mM NaCl and incubation was continued for an additional 20 min. After this, aliquots of particles to be used for transfer into cells were subjected to DNase treatment and gradient purification. To establish the level of DNA protected by the particles, the packaging mixture was adjusted to 10mM $MgCl_2$ and exposed to DNaseI (100 µg/ml) for 20 minutes at 30°C. An excess of calf thymus DNA and EDTA to 50mM was added, and packaged DNA was released from the pseudocapsids in the presence of 100mM dithiothreitol, 50mM EDTA and 1% w/v SDS. It was deproteinised by phenol: chloroform (24:1) extraction and loaded onto an agarose gel. The amount of input DNA fragment was identified by Southern blotting using a $^{32}$P-radiolabelled *Bcl*I-*Eco*RI fragment.

Data with regard to packaging are given in figure 1. Figure 1A contains a schematic diagram of the linear DNA fragment used in transfer in our initial studies and figure 1B shows an electron micrograph of the purified icosahedral VP1 particles utilised. The DNA was packaged into the particles by a previously described procedure, where viral empty capsids obtained during the course of a lytic polyoma virus infection were used (Barr *et al.,* 1979). Figure 1C shows a Southern blot of total input DNA (tracks 1 and 3) compared with that remaining after treatment of the packaging mixture with DNase I in the absence (track 2) or presence (track 4) of the pseudocapsids. About 2-5% of the input DNA was found to be incorporated as DNaseI-resistant material into VP1 pseudocapsids (compare tracks 3 and 4, figure 1C). In a complementary study (not shown), where radiolabelled DNA was packaged into pseudocapsids, about 5 % of the radioactivity was found to co-sediment with the particles on sucrose density gradient centrifugation.

**Example 2(i):** *Dissociation/Reassociation packaging of exogenous material.*

An alternative packaging method is to dissociate the capsids in the presence of an ion chelator, such as EGTA, and a reducing agent, such as beta-mercaptoethanol. (The concentrations of each chemical being calculated by titration for each capsid preparation.) The pseudocapsids are then reassociated, in the presence of the material being packaged, by dialysis against buffer A using a calcium ion concentration titrated for against the particular pseudocapsid preparation. Packaged material and particles can then be separated from unpackaged material by centrifugation through a 10-30 % sucrose gradient (made in buffer A, lacking glycerol) at 150000 x g for 3h.

**Example 3:** *Cellular response to introduced exogenous DNA.*

Following an encapsidation step, the packaging mixture was used to introduce DNA into recipient rat immortalized (rat-2) cell using a normal polyoma virus infection protocol. Three quarter confluent cell sheets were washed once in culture medium, DMEM, lacking serum, and pseudocapsids, (diluted to 1ml/$10^6$ cells with DMEM) were added. The cells were incubated at 37°C for 90 min and then supplemented with 4ml DMEM + 10% foetal calf serum. Cells were left in culture to observe whether dense foci, a marker of MT expression and cellular transformation, would develop. Control experiments were performed on rat-2 cells using the same amount of DNA employed in the initial packaging step either in mock infection (in the absence of pseudocapsids) or in co-precipitation with calcium phosphate in a classical transfection procedure (Graham and van der Eb, 1973). In these experiments, dense foci of cells were visible after three weeks in all but the mock-infected cultures. Foci were either counted after staining or taken from the plate for propagation in culture. The data, given in Table 1, are results from two experiments. They show that the numbers of foci obtained with pseudocapsids considerably exceed those from the calcium phosphate transfection. If the relatively low efficiency of the packaging step (see figure 1C) is taken into consideration, in assessing the numbers of dense cell foci produced in the respective experiments, the pseudocapsid route of DNA introduction was found to be 50-100 fold more effective than the transfection procedure for delivering functionally useful DNA to the cells.

Table 1.

| Comparison of Efficiency of Formation of Dense Foci. | | |
|---|---|---|
| Method of DNA transfer | No. of foci per dish | Efficiency of transfer (foci/microgram DNA) |
| Absorption of "naked" DNA | 0 | 0 |
| Calcium phosphate transfection | 15 | 7.5 |
| Pseudocapsid transfer | 57 | 570* |

\* Calculation based on packaging efficiency of 5 % .

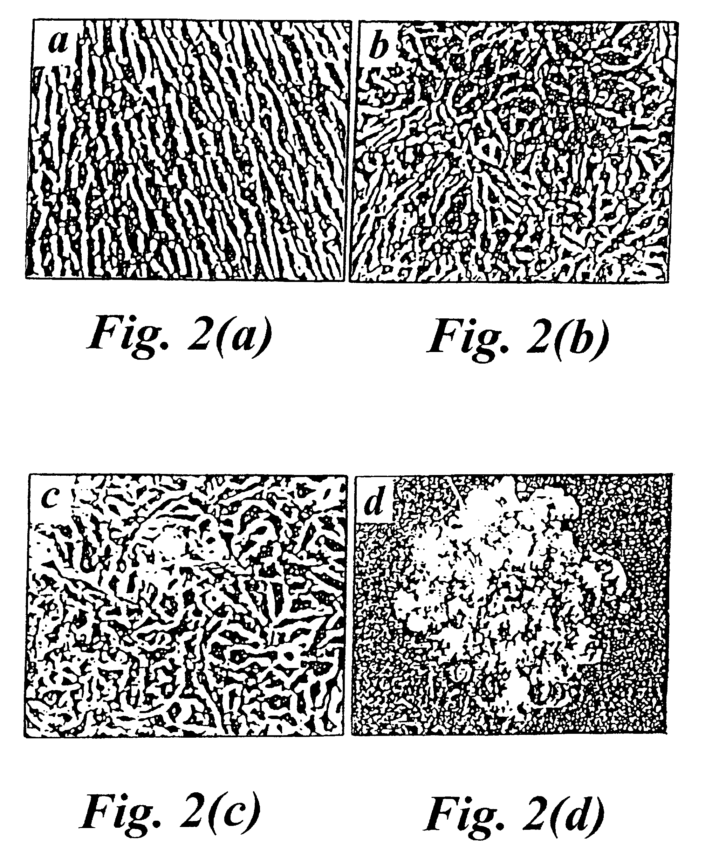

Individual foci, picked from the transformation assays and established in monolayer culture, were compared morphologically with the initial rat-2 cultures and assessed for their ability to grow as colonies in semi-solid media, another marker of cellular transformation. Figure 2 shows the morphology of the normal (mock infected) rat fibroblast cells (Figure 2 a) compared with those of a typical transformed cell culture established from foci selected from calcium phosphate transfected (Figure 2 b) or pseudocapsid treated cells (Figure 2 c), as well as typical colony growth observed in soft agar culture (Figure 2 d) using cells shown in Figure 2 c. The formation of transformed colonies suggested that the *dl8*MT gene packaged into VP1 particles produced by the recombinant virus had been stably introduced into the rat-2 cells, and is apparently functionally active.

To demonstrate that the latter was the case, rather than, for example, transformants having arisen spontaneously, protein samples were analysed for the presence and expression of the viral antigen. Two assays were used to examine MT in these cell lines, one involving immuno (Western) blotting, and the other examining the well-characterized interaction between MT and the cellular oncogene, *c-src* (Courtneidge and Smith, 1983), using a standard protein kinase assay (Collett and Erikson, 1978). The Western blot data (figure 3A) show that *dl8*MT - which migrates by SDS-gel electrophoresis faster than wild type MT, due to a deletion in the gene - was expressed in either transfected or pseudocapsid-treated rat-2 cells, and that the levels of expression are high (tracks 1-3) when compared with that found in equal numbers of *dl8* virion-derived transformed cell line (track 5). (In the latter case, the large T-antigen, which cannot be expressed off the recombinant *dl8* MT cDNA shown in figure 1a, is also observed, as was the viral small T-antigen, not shown.) Similar data (not given) were obtained from the other foci-derived cell lines. The results obtained from an *in vitro* kinase assay (figure 3B) are consistent with those from focus formation and soft agar assays. They demonstrate that the MT produced after pseudocapsid treatment of cells is able to interact with, and activate, the product of the cellular *src* oncogene in a manner indistinguishable from MT produced in virally-infected cells (Smith *et*

*al.,* 1979; Courtneidge and Smith, 1983), and thus appears to be fully functional. No MT activity was observed in the rat-2 parental cells.

### State of exogenous DNA in host cells.

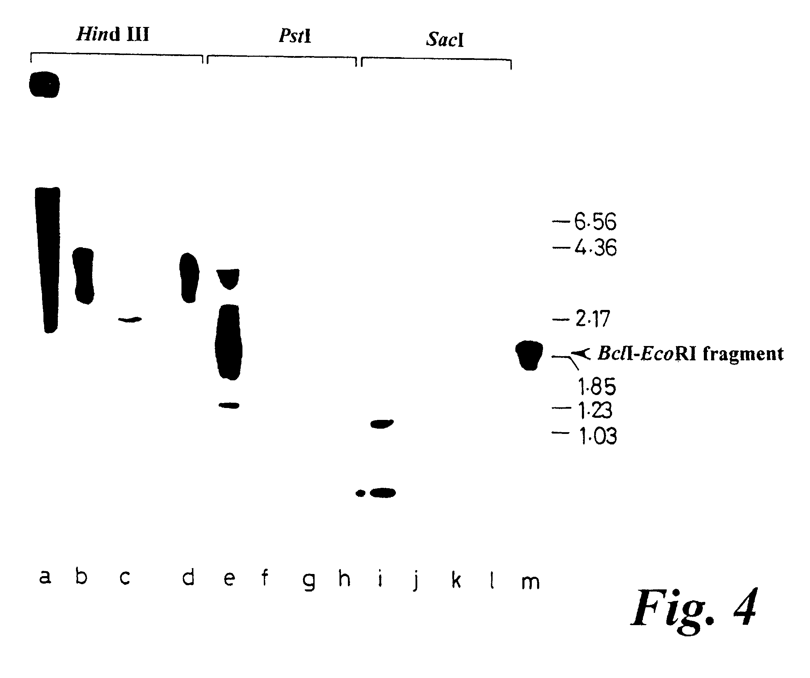

After showing efficient and biologically active expression of the *dl8*MT gene from DNA transported into cells *via* pseudocapsids, the inventors examined the state of the exogenous DNA in the cell, with particular regard to whether or not it had been integrated into the host chromosome. Southern blot analyses of total cellular DNA from early passages of two lines of cells derived from pseudocapsid transfer, and one from calcium phosphate transfection, were thus carried out, alongside the corresponding DNA from the parental (rat-2) cells. The data obtained are presented in figure 4. Here it can be seen that calcium phosphate transfected cultures contain high copy numbers of non-(or tandemly-integrated *dl8* DNA, as illustrated (tracks a, e and i), whereas much lower levels, if any, of these forms of DNA are observed in the experiments where the polyoma VP1 pseudocapsids were used for gene transfer (tracks b, c, f, g, j and k). In the latter case, bands are present on the gels that probably reflect single copy integration into the host chromosome suggesting that the incoming DNA is incorporated into the host genome probably at one, or a few sites only. To assess this further, DNA was cleaved with appropriate restriction enzymes (see figure 1A). Digestion with *Sac*I resulted in the release of an internal 654bp viral fragment. This band is clearly evident in DNA from the calcium phosphate transfected cells, and visible on longer exposures in lines generated from the VP1 pseudocapsid transfer (figure 4, tracks i-k), suggesting that the incorporated viral DNA has not undergone gross rearrangement. These data also demonstrate that the high level of *dl8*MT expression observed (figure 3) cannot be linearly related to copy numbers of the incorporated DNA since cells derived from the calcium phosphate precipitation technique (tracks a, e and i) contain multiple copies of the introduced gene, whereas pseudocapsid transfer results only in low level or single integration events (tracks b, c, f, g, j and k).

### Example 4: *Comparison of DNA carrier systems*

Semi-confluent 25 cm$^2$ T-flasks of rat-2 immortalized cells were either mock infected by exposure to 1μg of the "naked" *Bcl*I-*Eco*RI linear DNA fragment containing the gene for polyoma MT, or transfected with 1μg of this fragment using calcium phosphate, or treated with 60μg of pseudocapsids mixed with 1μg DNA, respectively. Media on cultures were changed weekly. At the end of three weeks, foci of transformed cells were either visualized for counting with Leishmann's staining solution or selected as individual foci for propagation. Cell lines from four foci (P1-4) obtained from pseudocapsid DNA transfer and from two foci (C1-2) isolated from calcium phosphate transfected cells were established in short-term culture. Individual isolated cell lines were analysed for their morphological appearance and their ability to grow as transformed cells in soft agar (Türler and Beard, 1985).

### Protein Analysis.

For Western immunoblot detection, proteins from total cell lysates were separated by SDS-PAGE (Laemmli, 1970), electroblotted and probed with monoclonal antibodies to polyoma virus early antigens (Dilworth and Griffin, 1982). Immunocomplexes were detected with a horseradish peroxidase conjugated secondary antibody (Dako plc) and visualised with ECL reagent (Amersham plc). Kinase assays were performed on immunoprecipitates with radiolabelled [$^{32}$P]- γ ATP (Collett and Erikson, 1978; Dilworth, 1982) using antibodies- Pab 701 and 721. Gels were exposed to autoradiographic film (XAR-5, KODAK) for four hours at -70°C.

### Southern blotting analyses.

Restriction enzyme digests of 20μg total cellular DNA were separated on a 0.8 % agarose gel, blotted and hybridised with $^{32}$P-labelled *Bcl*I- *Eco*RI DNA fragment. The filter was exposed to autoradiographic film (XAR-5, KODAK) for four days at -70°C.

### Example 5: *Introduction of heterologous DNA into human cells*

To investigate whether the papovavirus pseudocapsid carrier procedure could be adapted for human cells, the inventors sought to enhance the expression of an endogenous function, p43, found at low levels in normal cells (Koch *et al.,* 1990), by gene transfer into human embryo lung (HEL) fibroblasts. HEL cells, derived from primary human embryo lungs, had been passaged (weekly) about 30 times in culture. An agarose gel-purified *Nru*I/*Eco*RI restriction fragment (2.5μg) of the cellular gene, p43 (Koch *et al.,* 1990), cloned into pcDNA3 (Promega Inc) under the control of the cytomegalovirus immediate early promoter was packaged into VP1 pseudocapsids (15μg) as described above.

The packaged mixture, prepared in duplicate, was used to treat human embryo lung (HEL) fibroblast cells, using 2 x $10^5$ cells. As a control, cells were also treated with equivalent quantities of "naked" DNA, empty pseudocapsids, or with DNA transfected using the calcium phosphate co-precipitation technique. Cells were harvested 72 hr. later by washing with phosphate buffered saline (PBS), and lysing in Laemmli sample buffer (Laemmli, 1970). Proteins were fractionated by 10% SDS-PAGE, immunoblotted and probed initially with a p43 specific monoclonal antibody, as above, then anti-β tubulin antibody (Sigma). Immune complexes were detected with an HRP conjugated secondary antibody (Dako, plc) and chemiluminescent ECL reagent (Amersham plc). The results obtained from duplicate experiments are given in Figure 5A. They show a small (several-fold) but significant increase in gene expression in HEL cells treated with the pseudocapsids (tracks 1 and 4), as compared with that observed in mock or control experiments (cells treated with naked DNA or pseudocapsids only, tracks 2 and 3, respectively). Co-precipitation of the p43 gene with calcium phosphate (tracks 4 and 5) caused no increase in expression over basal level p43 expression. To establish the validity of these findings, the levels of tubulin in the experiments was assessed on the same gel. Significant differences were not observed in the quantities of tubulin seen in individual tracks when an anti-tubulin antibody was used, as shown in Figure 5B.

The results demonstrate that papovavirus pseudocapsids can be used as carriers for introducing biologically functional exogenous DNA stably into recipient cells. The pseudocapsids do not appear toxic to cells, in that little cell death was observed during the course of the experiments, even though immunofluorescence data (not shown) demonstrated high levels of pseudocapsid in many cells. In a comparison made with the calcium phosphate co-precipitation transfection method, the pseudocapsid carrier approach was found to be both more selective and more efficient. This may reflect either greater efficiency of DNA uptake, enhanced protection of DNA, more effective transport to nuclei, preferential integration into host chromosomes, or a combination of two or more of these factors.

These results show that at least transient up regulation of expression of the product of a human gene, p43 (Koch *et al.*, 1990), can be obtained in HEL cells when it is introduced by pseudocapsids. Thus, carrier papovavirus pseudocapsids have application in human cells. It is noteworthy that companion experiments using DNA co-precipitated with calcium phosphate (Graham and van der Eb, 1973) were not equally successful. Documentation in the past has noted the difficulties of transfecting "foreign" material into human cells (Hoeijmakers, *et al.* 1987; Mayne *et al.,* 1988). The corresponding success of the pseudocapsid carrier approach may rely on the fact that efficient targeting to the cell nucleus occurs by this procedure.

### *Increasing the amount of genetic material incorporated in VP1 pseudocapsid*

There is probably a size constraint on the amount of genetic information that might be packed within VP1 icosahedral pseudocapsids, and the upper limit for studies such as those performed here may be determined by the size (5.2kbp) of the polyoma wild type viral genomes. Using the passive polyoma packaging procedure of Barr *et al.* (1979) or Slilaty *et al.* (1982), using "naked" DNA, the limit appears nearer to 3kbp. Whereas this size would be adequate for expression of many mammalian cDNAs, or oligonucleotides to be used for antisense "knockout" experiments, or other small biologically active molecules, it would preclude packaging of a number of genes in their entirety. For gene therapy purposes it may not, however, be necessary for the DNA to be fully encapsidated by the pseudocapsid.

### Example 6: *Associating pseudocapsids and DNA*

(i) The following technique can be used to increase the size of the DNA fragment or other exogenous material, which can be transferred to host cells by pseudocapsids. The technique involves packaging the DNA into empty pseudocapsids as described in Example 2 and then further protecting the DNA by incubation at 37°C for 20 mins in the presence of 10μg polylysine (pL300)/60μg pseudocapsids.

**(ii) *Alternative DNA packaging and pseudofection with pseudocapsids***

DNA was packaged into pseudocapsids according to the procedure of Barr *et al* (1979). For *in vitro* packaging, approximately 1μg of circular or linear DNA, containing the reporter gene, was mixed with 30μg of pseudocapsids and incubated at 37°C for 10 minutes. The mixtures was exposed to osmotic shock by dilution with 4 volumes of $H_2O$. In experiments where polylysine (Sigma; mol. wt. 30,000-70,000) was used as a means of condensing DNA, 2μg of polylysine was added to the DNA/psuedocapsid mixture before osmotic shock; the reaction was incubated at 37°C for a further 10 minutes, then added to cells. Half confluent cell cultures were incubated for a further 90 minutes with occasional rocking, then supplemented with excess DMEM medium supplemented with 10% FCS.

*Protection against DNaseI of unencapsidated exogenous DNA with polylysine*

pCMVβ DNA (1μg) was used for assessing the protection of unencapsidated material by pseudocapsids in the presence or absence of polylysine. Following a packaging step[1], 0.11 U pancreatic DNaseI was added to the packaging mixture in the presence of 1mM $MgCl_2$ and 0.5 mM $CaCl_2$ and the solution was incubated for 60 minutes at 37°C. The enzymic reaction was stopped by the addition of a solution consisting of 768 mM sodium acetate, 128 mM EDTA and 256μg/ml yeast RNA. To release packaged DNA from pseudocapsids, the reaction mix was digested in proteinase K at 37°C for 20 minutes. Trypsin/versene was used to separate DNA from polylysine. The solutions were extracted with an equal volume of phenol-chloroform (25:24 vol/vol) and the DNA precipitated with cold ethanol, resuspended in TE (pH 8), loaded onto a 1.0% agarose gel containing ethidium bromide and subjected to electrophoresis.

*Analysis of the interaction of DNA, pseudocapsids and polylysine*

To mixtures of DNA and VP1 packaged in the presence of polylysine in Buffer B, adjusted to 2.5g in weight, 1.2g of CsCl was added, and the final solution transferred to an ultracentrifuge tube (Beckman rotor SW 55). Pseudocapsids containing DNA were separated from empty pseudocapsids by centrifugation in an SW 55 rotor at 40,000 rpm for 20 hours at 20°C. The resulting gradient was collected in 0.5ml fractions, 10μl of each fractionated sample was treated with Proteinase K (0.1μg) at 37°C for 20 minutes in the presence of 1% SDS. After phenol/chloroform extraction, DNA was boiled for 5 minutes, and cooled on ice. Samples were applied to a presoaked (in 2 X SSC) membrane (Zeta-Probe, or Biodyne B. Pall), using a slot blot apparatus, and hybridized with a pCMVβ (digested with EcoRI) $^{32}$P-labeled probe made according to the instructions of the multiprime DNA labelling kit (Amersham). Membranes were exposed to X-ray film (XAR-5, Kodak) for 20 minutes at room temperature, and the resulting hybridization signals analysed by densitometry.

*Histochemical staining for ß-galactosidase activity in cells*

Cells transfected with combinations of DNA and pseudocapsid, with or without polylysine, were washed with PBS and fixed in 0.1 % glutaraldehyde in PBS for 10 minutes at room temperature, then washed twice in PBS, and incubated in X-gal solution (5mM $K_3Fe(CN)_6$, 5mM $K_4Fe(CN)_6$:$3H_2O$, 2mM $MgCl_2$, 200μg/ml 5-bromo-4-chloro-indolyl-β-D-galactopyranoside in N-N-dimethylformamide) for 20-24 hours at room temperature. Expression of the β-galactosidase gene product in cells was detected microscopically by an indigo blue colour.

*Staining for ß-galactosidase activity in whole tissues*

Whole tissues from mice, immediately following dissection, were washed in PBS and fixed with a solution consisting of 1% formaldehyde, 0.2% glutaraldehyde, 2mM $MgCl_2$, 5mM EGTA, 0.02% NP-40. Excess fixative was removed after 1 hour by washing in PBS, and the sample was incubated overnight in the dark with staining reagents (5mM $K_3Fe(CN)_6$, 5mM $K_4Fe(CN)_6$:$3H_2O$, 2mM $MgCl_2$, 0.02% NP-40, 1mg/ml X-gal [5-bromo-4-chloro-3-β-D-galactopyranoside, Promega, Madison Wisc, USA]) in PBS. All procedures were carried out at room temperature. Samples were examined by low power light microscopy and areas of blue stain scored in whole tissues, alternatively for confirmation, sections (1-2mm) were excised from areas of stained tissue and observed at high magnification.

*RNA isolation and reverse transcriptase-(RT) PCR assays*

Total RNA was isolated from either a transfected or pseudofected (with pseudocapsids) mammalian cell culture (rat 2 cells) using an RNA extraction kit (QIAGEN). Isolated total RNA was digested with DNase1 to remove DNA. Synthesis of cDNA was performed using an oligonucleotide reverse primer (5'-TTCCAGATAACTGCCGTCACTCC). To inactivate reverse transcriptase and denature the RNA/cDNA hybrids, samples were incubated at 96° C for 5 minutes. PCR was carried out with an oligonucleotide primer (5'-GGCATTGGTCTGGACACCAGCA) and the reverse primer in a thermal cycle (Hybaid) using a programme consisting of one cycle of 5 minutes at 94°C, 45 seconds at 60°C and 2 minutes at 72°C, followed by 30 cycles of 45 seconds at 94°C, 45 seconds at 60°C and 2 minutes at 72°C. One final cycle of 10 minutes at 72°C completed the reaction. PCR products were analysed by electrophoresis in a 1.0% agarose gel and the products were transferred to a Hybond N (Amersham) nylon membrane and hybridized with a $^{32}$P-labelled pCMVβ DNA probe (see above).

To overcome the apparent limited DNA size capacity of pseudocapsid carriers, and protect non-packaged DNA from degradation during its route through the cell, as well as to condense the complexes, we explored the use

[1]Forstova, J. *et al.* Polyoma virus pseudocapsids as efficient carriers of heterologous DNA into manunalian cells. *Hum Gene Ther;* **6**: 297-306 (1995).

of the polycationic amine, poly-L-lysine, for accomplishing these aims. Polylysine has been successfully used both in receptor-mediated gene transfer and as a means of linking heterologous DNA to specific targeting ligands for other receptor-mediated approaches in gene transfer experiments[2,3]. An electrostatic interaction between the negatively charged DNA and the positively charged polymer serves not only to bind the DNA to ligands but also to condense it into a compact structure [4]. Polylysine has also been used together with adenovirus vectors to enhance efficiency of uptake of the latter[5].

Our studies show that polylysine, added to the DNA/pseudocapsid transfer system, augments short-term delivery of gene activity to cells, including human, *in vitro,* and has similar effects in *in vivo* experiments. Its presence is not, however, mandatory for transferring higher molecular weight DNAs by pseudocapsids, and may even be of negative value for long-term gene expression. Transgene expression *in vivo* by the pseudocapsid routes was sustained for several months without detectable pathology, with some differences observed with regard to targeted organs when polylysine was present. Thus, whereas polylysine *per se* alters the ability of VP1 pseudocapsids to enter cells specifically and enhances transient expression, it may be of limited value for long-term expression of exogenous genes.

### *Protection of unencapsidated exogenous DNA with polylysine*

The major polyoma viral capsid antigen, VP1, self-assembled into pseudocapsids, has the ability to bind DNA non-specifically in a cell-free system. In the original studies, the size of exogenous DNA found in association with pseudocapsids and protected from low level co-purifying cellular DNaseI activity, was found to be about 3 kbp[6]. We have subsequently modified the purification protocol of VP1 in such a way as to remove accompanying DNase1 activities. In attempts to increase the efficiency of transfer of larger DNAs, we tested whether polylysine could interact with DNA/pseudocapsid complexes, and measured the degree of protection it afforded against the action of cellular DNases *in vitro* or *in vivo.* Data using a 7.2 kbp supercoiled plasmid DNA (pCMVβ) are shown (Figure 12). Digestion by DNaseI of plasmid DNA in DNA/pseudocapsid complexes, with or without added polylysine, was analysed by gel electrophoresis. The results shown that most of the DNA packaged with pseudocapsids, without polylysine protection, was degraded to about 3 kbp in size (lane 2), consistent with earlier work[6]. With DNA mixed with polylysine, followed by addition of VP1 pseudocapsids, most of the DNA was protected (compare lane 3 with lane 1), as was the case when polylysine was added to pre-formed DNA/pseudocapsid complexes (compare lane 4 with lanes 1 and 3). In both cases, the products differed from the initial DNA (lane 3) in being mixtures of supercoiled, circular and linear DNAs, suggesting that polylysine *per se,* may result in DNA nicking. Similar data (not shown) were obtained with polylysine in the absence of pseudocapsids.

### *Structural alterations influenced by the mode of complex formation*

Following packaging, interactions of either DNA/pseudocapsids, DNA/polylysine, or mixtures of all three (DNA/polylysine/pseudocapsids) were analysed by observing the mobility of complexes by CsCl equilibrium centrifugation. Complexes of pCMVβ (7.2 kbp) DNA with either pseudocapsids, polylysine, or both reagents, were sedimented on CsCl gradients; the location of pseudocapsids was detected by measurement of refractive indices and identification of peak VP1 fractions by SDS-PAGE. An aliquot of each fraction was transferred onto filters by slot blotting and the DNA identified by hybridization with $^{32}$P-randomly labelled linearised pCMVβ DNA. The strength of the signal in each fraction was determined by densitometric analysis of the resulting autoradiograms. The data obtained are given in Figure 13: Uncomplexed DNA sedimented at the bottom of the CsCl gradient, where DNA complexed with polylysine only was observed in fractions with refractive indices between that of DNA and pseudocapsids (Figure 13, compare panels A and B). Pre-formed DNA/pseudocapsid complexes, mixed with polylysine (panel c) were more prominent in fractions with the density of VP1 pseudocapsids, whereas DNA bound first with polylysine, followed by addition of pseudocapsids (panel D), showed little comigration with pseudocapsids.

As pCMVβ DNA mixed with polylysine before treatment with pseudocapsids appeared to form different structures from those observed when the mixing protocol was reversed, complexes were further studied by electron microscopy (EM), using materials produced under conditions identical to those described above. The reaction mixture of pCMVβ DNA and pseudocapsids after packaging (Figure 14, panel A) contained complexes consisting of two, three or four particles per DNA molecule, whereas in the presence of polylysine, profound condensation of DNA occurred

[2] Wagner, E. *et al.* Polyomavirus major capsid protein VP1 is capable of packaging cellular DNA when expressed in the baculovirus system. *J. Virol.* **71**: 2857-2865 (1997).

[3] Cotten, M *et al.* High-efficiency receptor-mediated delivery of small and large (48 kilobase) gene constructs using the endosome-disruption activity of defective or chemically inactivated adenovirus particles. *Proc. Natl. Acad. Sci. USA;* **89**: 6094-8 (1992).

[4] Wagner, E., Gotten. M., Forsner, R. and Bimstiel, M.L. Transferrin-polycation-DNA complexes: the effect of polycations on the structure of the complex and DNA delivery to cells. *Proc. Natl. Acad Sci. USA;* **88**: 4255-9 (1991)

[5] Arcasoy, S.M. *et al.* Polycations increase the efficiency of adenovirus-mediated gene transfer to epithelial and endothelial cells *in vitro. Gene Therapy;* **4**: 32-38 (1997).

[6] Forstová J. *et al.* Polyoma virus pseudocapsids as efficienct carriers of heterologous DNA into mammalian cells. *Hum Gene Ther;* **6**: 297-306 (1995).

(panel B). DNA/pseudocapsid complexes were further condensed as a result of subsequent addition of polylysine (panel C). Pseudocapsids mixed with DNA/polylysine complexes produced gross aggregation with many molecules of DNA and capsids combining with polylysine (panel D). Structural alterations seem likely to influence DNA uptake by cells, and the considerable bulk of a complex, such as seen (panel D) might be expected to inhibit transfer of material to the cell nucleus for long-term expression, particularly if uptake is receptor-mediated[7].

To test this supposition, the appropriate complexes of DNA/polylysine, DNA/pseudocapsids, or DNA mixed with polylysine and pseudocapsids, were applied to human CCL 13 cells, as described (Materials and Methods). In preliminary experiments, cells were left for 48 hours and expression of the β-galactosidase reporter gene identified by a histochemical assay (staining with Xgal to produce blue stained cells) and quantified. Although the efficiencies were low, blue cells were observed in each experiment, with polylysine addition resulting in considerable augmentation of expression in each instance (data not shown). In subsequent experiments, as part of the aim of this work was to assess the role of pseudocapsids in gene transfer, ternary complexes were always generated by adding polylysine to pre-formed DNA/pseudocapsid complexes.

### Influence of the concentrations of polylysine and Ca$^{2+}$ ions on pseudofection efficiency

In an attempt to enhance efficiency, we explored whether varying the ratios between DNA/pseudocapsid complexes and polylysine might alter their structure, as suggested by the EM study, and influence the efficiency of pseudofection. To establish an optimal ratio of DNA/pseudocapsid complexes and polylysine in the reaction, constant amounts of DNA, or DNA/pseudocapsids, were mixed with increasing amounts of polylysine and mixtures were incubated with CCL 13 cells. Experiments were repeated on more than one occasion and the mean result is given in Figure 15A. Consistent augmentation of pseudofection was seen when DNA/pseudocapsid pre-formed complexes were condensed with polylysine at a concentration of 0.2μg/μl. Higher concentration of polylysine enhanced expression with naked DNA, but proved toxic for cells treated with DNA/pseudocapsid complexes. Based on these data, a polylysine concentration of 0.2μg/μl was taken to be optimum and was used subsequently for pseudofection reactions.

Ca$^{2+}$ is required for self-assembly and maintenance of VP1 pseudocapsids, and is known to play an important role in assembly, stability and disassembly of the outer capsid during the polyomavirus life cycle, affecting the ability of capsids to penetrate the cell membrane[8]. In order to find the optimal concentration of calcium ions for efficient gene transfer to CCL 13 cells using pCMVβ DNA/pseudocapsids complexed with polylysine, pseudocapsids were isolated in buffers with differing (0μM, 5μM, 50μM and 100μM) concentrations. As shown in Figure 15B, in the presence of polylysine, DNA complexed with capsids in low [Ca$^{2+}$] buffer showed significant levels of gene transfer. Consistent enhancement of gene transfer was observed with pseudocapsids prepared in a buffer containing 10μM Ca$^{2+}$.

### Persistence of ß-galactosidase expression in vitro

Having progressed towards optimising transient pseudofection efficiencies, at least for pCMVβ DNA, persistence of β-galactosidase activity was examined in cells transduced with various preparations of complexes. For each complex, cultures of CCL 13 cells were pseudofected with DNA/pseudocapsid complexes, with or without polylysine, or, as control, transfected with DNA mixed with polylysine. Pseudofected cells were maintained up to 21 days (by subculturing at a 1:5 dilution every 3 days). Samples were taken at 2, 5, 11, 14 and 21 days, and stained for β-galactosidase expression with X-gal. After five days, the number of blue staining cells observed (about 400/well) was diminished by more than half in samples treated with DNA/polylysine or DNA/pseudocapsid complexes in the presence of polylysine. However, only a few stained cells were observed after 11 days, and none after 14 days (data not shown). With the relatively insensitive histochemical assay, it was not obvious that long-term expression of large DNAs, in cells that require extensive recycling, occurred by any of the chosen routes.

The expression of β-galactosidase in rat 2 cells, which remain viable in static culture, was thus similarly tested, and blue cells (diminishing in number) were observed on days 2, 14 and 21 following either pseudofection, or transfection of DNA in the presence of calcium phosphate.

Quantitation of data was difficult in these cells, however, as expression of the endogenous enzyme resulted in high in background staining, using any protocol.

As an alternative assay, expression of β-galactosidase was evaluated at the mRNA level by the reverse transcription-polymerase chain reaction (RT-PCR) using total RNA and oligonucleotide primers for the bacterial β-galactosidase gene. The data, given in Figure 16, show expression over time in cells treated with DNA/pseudocapsid complexes in the absence (tracks 7-9) or presence (tracks 10-12) of polylysine. Total RNAs, purified from rat 2 cells 2 days

[7] Zenke, M. *et al.* Receptor-mediated endocytosis of transferrin-polycation conjugates: an efficient way to introduce DNA into hematopoietic cells. *Proc Natl Acad Sci* USA; **87**: 3655-9 (1990).

[8] Brady, J.N., Winston, V.D. and Consigli, R.A. Dissociation of polyoma virus by the chelation of calcium ions found associated with purified virions. *J. Virol;* **23**: 717-24 (1977).

after transfection with DNA alone without (tracks 4-6) or in the presence (tracks 13-15) of polylysine, or in the presence of calcium phosphate (tracks 16-18), were used as controls. Treatment of cells with DNA/pseudocapsids (pcaps) only, resulted in moderate expression of the β-galactosidase RNA immediately following pseudofection, with expression declining to a lower, but stable, level with time (tracks 7 to 9). Addition of polylysine (pLK) augmented the initial levels of the β-galactosidase transcript (compare, for example, track 10 with 7), in a manner that also decreased in intensity with time (tracks 10 to 12), the long-term effect being similar to that seen in the absence of pseudocapsids. When polylysine alone, or calcium phosphate, were used as transfection agents (tracks 13 to 15 and 16 to 18, respectively), a much higher initial level of transcription was achieved, but this rapidly declined, not appearing, particularly in the latter, to reach a steady state by 21 days. These data are consistent with previous *in vitro* experiments where pseudocapsids were seen to favour long-term over short-term expression[9].

### *Persistence of ß-galactosidase gene expression in vivo*

We queried whether matrices, laid down in order to allow cells to adhere to plastic, might influence the data obtained in *in vitro* experiments. To circumvent *in vitro* artefacts, *in vivo* experiments in mice were carried out to assess the effects of polylysine on pseudofection. Pseudocapsid/DNA complexes, in the presence and absence of polylysine, using the 7.2kbp pCMVβ DNA as reporter gene (or as controls naked DNA) were injected in nude mice subcutaneously. Tissues were collected at 1 and 7 weeks after injection and expression of the β-galactosidase gene was assessed by a histochemical assay. The data are summarised in Table 2A, where "high" and "intermediate" expression indicates the relative levels of blue coloration observed histochemically. "Low or no expression" indicates apparently negative results. After 1 week, high level expression of the β-galactosidase gene was observed in the spleen, kidney and brain of mice injected with DNA/polylysine only, or with polylysine complexed to DNA/pseudocapsids. With the latter, the skin from mice also showed strong gene expression at the site of injection. Mice injected with DNA/pseudocapsid complexes showed high expression only in the spleen. Seven weeks after injection, as in the longer-term *in vitro* experiments, the expression pattern had changed. High level expression was seen in the heart of mice injected with DNA/pseudocapsid complexes, and intermediate expression was found in spleen, kidney and brain, as reported[10]. Moderate expression could still be detected in some organs of the mice injected with DNA/pseudocapsid complexes in the presence of polylysine. However, most gene expression had been lost from mice injected with DNA or DNA/polylysine complexes only. These results support the suggestion that VP1 pseudocapsid complexes favour long term *expression in vivo* as well *as in vitro.*

In the above studies, poly-L-lysine (polylysine), widely used for receptor mediated gene transfer[11,12,13,14] was added to protect DNA that was not incorporated in the pseudocapsid complexes, to facilitate gene transfer; this reagent improved the levels of protected DNA when greater than polyoma genome-sized material was used (see Figure 12). When exploring effects of the temporal order on creating complexes, polylysine mixed first with DNA, then added to pseudocapsids, was found to result in large aggregates (Figures 13D and 14D). Subsequent experiments, therefore, used a protocol involving initial mixing of DNA and pseudocapsids, followed by polylysine addition, to avoid such aggregates. To optimise the procedure, varying concentrations of $Ca^{2+}$ or polylysine were assessed. DNA/capsid/polylysine complexes gave best expression of the β-galactosidase gene *in vitro* using 0.2µg/µl of polylysine during complex formation; DNA/polylysine complexes alone performed best with higher concentrations. Electronmicroscopy data (Figure 14) suggest that polylysine condenses the DNA/pseudocapsid complexes. For $Ca^{2+}$, which plays a critical role in viral capsid assembly, disassembly, and penetration of cell membranes[15,16] a concentration of 10µM proved optimum (Figure 15B); interestingly this is the concentration in buffers used for purification of genuine polyoma capsids and virions [17].

Since sustained long-term expression is a desirable component of gene therapy[18], we attempted to assess this property in tissue culture, monitoring β-galactosidase gene expression from a 7.2kbp recombinant DNA in CCL

[9] Forstová J. *et al.* Polyoma virus pseudocapsids as efficient carriers of heterologous DNA into mammalian cells. *Hum Gene Ther;* **6**: 297-306 (1995).

[10] Krauzewicz, N. *et al. In vivo* gene transfer by polyoma pseudocapsid protein assemblies *manuscript in preparation.*

[11] Wagner, E. *et al.* Transferrin-polycation conjugates as carriers for DNA uptake into cells *Proc Natl Acad Sci USA:* **87**: 3410-4 (1990).

[12] Wagner, E. Cotten. M., Forsner, R. and Birnstiel, M.L. Transferrin-polycation-DNA complexes: the effect of polycations on the structure of the complex and DNA delivery to cells. *proc Natl Acad Sci USA;* **88**: 4255-9 (1991).

[13] Arcasoy, S.M. *et al*, Polycations increase the efficiency of adenovirus-mediated gene transfer to epithelial and endothelial cells *in vitro. Gene Therapy;* **4**: 32-38 (1997).

[14] Zenke, M. *et al.* Receptor-mediated endocytosis of transferrin-polycation conjugates: an efficient way to introduce DNA into hematopoietic cells. *Proc Natl Acad Sci USA;* **87**: 3655-9 (1990).

[15] Brady, J.N., Winston, V.D. and Consigli, R.A. Dissociation of polyoma virus by the chelation of calcium ions found associated with purified virons. *J Virol.* **23**: 717-24 (1997).

[16] Ruiz, M.C. *et al.* The concentration of $Ca^{2+}$ that solubilizes outer capsid proteins from rotavirus particles is dependent on the strain. *J Virol;* **70**: 4877-83 (1996).

[17] Turier, H. and Beard, P., in "Virology" (ed. Mahy, B.W.J.) (IRL Press, Oxford, 1985) pp. 169-192.

[18] Wagner, E. *et al.* Polyomavirus major capsid protein VP1 is capable of packaging cellular DNA when expressed in the baculovirus system. *J. Virol.;* **71**: 2857-2865 (1997).

13 human liver cells, adopting various modes for gene delivery. The data obtained were consistent with a level of gene expression that, in the short term, was enhanced in the presence of polylysine, but gradually declined on continuous culture (with or without pseudocapsids) below histochemically detectable levels. To examine the possibility that pseudocapsids require time to achieve efficient gene delivery, rat 2 cells, which do not require subculturing to survive for about 3 weeks, were assessed by the same experimental routes used for CCL 13 cells, measuring transcriptional expression by RT-PCR. *In vitro* studies all pointed to similar conclusions. That is, whereas

DNA/polylysine complexes on their own were adequate for transient gene expression, the levels of expression were unstable, as others have found[19,20] and by 21 days were still declining (Figure 16, compare tracks 13-15). When pseudocapsids were present (tracks 10-12), there was less distinction between the 14 and 21 day experiments. The same was true for pseudofection experiments (tracks 7 to 9), although here the short-term transcriptional expression (track 7) was below that seen (track 10) in the presence of polylysine. The results point to cooperative effects between pseudocapsids and polylysine, and indicate that with our present protocol, polylysine enhances short-term expression, whereas pseudocapsids alone favour long-term, rather than high level expression.

To explore this topic further, we turned to animal experiments, where time would not present the constraints experienced with cells in tissue culture. Complexes, made as defined for the *in vitro* culture work were injected subcutaneously into mice and the results were investigated at 1 week (as representing short-term expression) and 7 week (for long-term). We found that at 1 week, generally similar results were obtained in the polylysine experiments, with or without pseudocapsids, with highest levels of expression being observed in spleen, kidney and brain. In the presence of pseudocapsids, without polylysine, targeting was mainly to the spleen. With DNA alone, the overall expression levels in all organs were lower, and with this route, by 7 weeks, no expression was observed. At this time, with DNA/polylysine complexes, the levels of expression had also declined. With DNA/pseudocapsid complexes, on the other hand, expression levels were sustained or even increased, a number of organs having been stably targeted including, unexpectedly, the brain. Interestingly, polylysine appears to allow targeting to the parotid gland, not seen with DNA/pseudocapsids on their own. The lack of efficiency in the early stages of delivery by pseudocapsids appear to be compensated for later, presumably by stable integration into the host chromosome. Note that relative to other transfer vector systems, small amounts (5µg) of DNA are used in each experiment involving pseudocapsids.

Gene transfer using pseudocapsids resembles non-viral gene transfer systems in that none of the viral genome is present. Our experiments were designed to study the role of polylysine in assisting gene transfer of high molecular weight DNA, having assumed that it (or a similar chemical agent) would be required to protect DNAs larger in size than those that could be protected by the pseudocapsids alone (that is, about 3kbp). Our current experiments show that this is not the case, and that VP1 pseudocapsids on their own can deliver a gene so as to produce sustained expression both *in vitro* and *in vivo*, encoded in larger plasmids such as the 7.2kbp DNA, used in most of our assays. (We have preliminary data that expression with plasmids larger than 10kbp in size can also be achieved by this route, unpublished observations.) Although polylysine was effective in increasing transient expression of an input reporter gene, it provided little apparent value in experiments aimed at long-term persistent expression. However, its use may be advantageous in directing the gene to a particular organ or cell type (see Table 2A) and in situations where levels of gene expression rather than duration are of prime importance.

**Example 7: An alternative approach is to precondense DNA with a binding protein prior to packaging.**

**Example 8: *Larger pseudocapsids***

An alternative approach to allow larger amounts of exogenous material such as DNA fragments to be transferred to host cells by pseudocapsids, takes advantage of the observation that not all the baculovirus expressed papovavirus major capsid antigen such as VP1 self-assembles into standard 45nm icosahedral structures. Rather, some of the capsid antigen produced assembles into much larger spherical and filamentous shapes (Forstová *et al.,* 1993). Similar morphological findings were reported earlier where, with polyoma virus infected cells, electron microscopic examination revealed capsids ranging in size from 27 to 65 nm (Eddy, 1969). In the present invention, the larger of these assemblies are isolated and used to package DNA as described in Examples 1 and 2. These larger pseudocapsids can package and transfer much larger DNA fragments than the standard sized icosahedral structures.

**Example 9: *Formation of pseudocapsids from HPV L1 protein***

Pseudocapids may be formed from human papillomavirus L1 protein by the method of Hagensee *et al* (1993) *J. Virol.* **67**, 315-322. Briefly, the HPV L1 gene (from HPV type 1 in this case) is inserted into a vaccinia virus by the

[19] Wagner, E. *et al.* Transferrin-polycation conjugates as carriers for DNA uptake into cells *Proc Natl Acad Sci USA; 87*: 3410-4 (1990).
[20] Wagner, E., Cotten. M., Forsner, R. and Bimstiel, M.L. Transferrin-polycation-DNA complexes: the effect of polycations on the structure of the complex and DNA delivery to cells. *Proc Natl Acad Sci USA; 88*: 4255-9 (1991).

TK recombination method and expressed in suitable cells, for example BSC-1 monkey kidney cells. Similarly, L1 can be expressed from a recombinant baculovirus.

Purification of HPV-1 capsids: BSC-1 cells (20- to 150-mm plates) were infected with recombinant vaccinia virus at a multiplicity of infection of 30. Cells were incubated at 37°C for 72 hr in Dulbecco's modified Eagle's medium supplemented with 5% fetal bovine serum and antibiotics. Cells were then collected by aspiration. Cells were pelleted by centrifugation (1,000 x g, 10 min) and washed once with phosphate-buffered saline (PBS). A nuclear fraction was prepared by resuspending the cell pellets in 10 ml of HEPES [N-2-hydroxyethylpiperazine-*N*'2-ethanesulfonic acid] buffer (10 mM HEPES [pH 7.9], 10 mM KCl, 1.5 mM $MgCl_2$) and incubating on ice for 10 min. Cells were lysed by homogenization, 50 strokes with a tight-fitting pestle. Nuclei were collected by centrifugation at 2,000 x g for 10 min. The nuclei were resuspended in 2 ml of PBS and sonicated for 2 min, and 2.1 g of cesium chloride was added. The nuclear suspension was placed into ultracentrifuge tubes, and the density was adjusted to 1.340 g/ml. Samples were spun at 45,000 rpm for 36 hr at 4°C in an SW55 rotor. Fractions were collected by puncturing the bottom of the tube or by needle aspiration of the capsid band. Density of the resultant fractions was determined with a Bausch & Lomb refractometer. Fractions were dialyzed against three changes of PBS, and HPV proteins were detected PBS', by Western blot. Fractions containing L1 proteins were analyzed for pseudocapsid formation by electron microscopy. The yield was approximately $10^{10}$ particles.

To determine whether the L1 proteins could self-assemble to form pseudocapsids, cells infected with the recombinant vaccinia virus were collected and nuclei were isolated, lysed, and layered onto cesium chloride gradients. After centrifugation for 48 hr, fractions were collected and analyzed for L1 protein by Western blot.

A large amount of L1 protein migrating at a density of approximately 1.30 g/ml was produced. When analyzed by electron microscopy, 55-nm icosahedral particles were found.

The empty pseudocapsids were associated with the exogenous material, for example a mammalian cDNA, by the osmotic shock and re-assembly techniques described above.

**Example 10:** *Association of empty pseudocapsids and other exogenous material.*

Non-genetic exogenous material such as proteins and polypeptides such as antibodies or fragments thereof, and pharmacologically active compounds, can be associated with empty pseudocapsids using the method described for exogenous DNA in example 2. The method is simply modified by substituting the exogenous material for the DNA.

It is preferred that the exogenous material is one which acts in or on the nucleus of a cell such as a nucleoside analogue. The pseudocapsids of the invention deliver material efficiently to the cell nucleus. For example, the pseudocapsids can be used to deliver hormones to the cell nucleus to trigger the action of nuclear signalling molecules which activate hormone responsive elements to control gene expression.

**Example 11:** *Administration of pseudocapsids to mammals.*

The aforementioned pseudocapsids of the invention may be administered by any conventional method including oral and parenteral (eg subcutaneous or intramuscular) injection. A preferred method of administration is by means of a nasal inhalation spray, in which the pseudocapsids are produced as in an aerosol. Such an approach has been used in the gene therapy of cystic fibrosis as an effective way of delivering the therapeutic agent to lung epithelial cells. The treatment may consist of a single dose or a plurality of doses over a period of time.

Whilst it is possible for a pseudocapsid of the invention to be administered alone, it is preferable to present it as a pharmaceutical formulation, together with one or more acceptable carriers. The carrier(s) must be "acceptable" in the sense of being compatible with the pseudocapsid of the invention and not deleterious to the recipients thereof. Typically, the carriers will be water or saline which will be sterile and pyrogen free.

**Example 12:** *Pharmaceutical formulations of the pseudocapsid*

The formulations may conveniently be presented in unit dosage form and may be prepared by any of the methods well known in the art of pharmacy. Such methods include the step of bringing into association the active ingredient (pseudocapsid of the invention) with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrate (eg sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide a desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostatis and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use.

Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

**Example 13(i): *Stable transfer of marker gene, β-galactosidase, to the central nervous system of the mouse.***

**METHODS**

*Isolation of pseudocapsids*

Sf9 insect cells infected with VL-VP1 recombinant baculovirus were harvested 72 hours post infection, resuspended in buffer A (150 mM NaCl, 10 mM Tris-HCl, pH 7.6, 10 $\mu$M CaCl$_2$, 5% glycerol, 0.01% Triton X-100) and disrupted by sonication at 15 micron amplitude in three 15 second bursts. Cell debris were removed by centrifugation at 8000 x g and pseudocapsids pelleted through a 20% sucrose cushion at 150,000 x g for 1 hour. The pellet was resuspended in buffer B (150 mM NaCl, 10 mM Tris-HCl, pH 7.6, 10 $\mu$M CaCl$_2$, 0.01 % Triton X-100) adjusted to 1.30-1.33 g/ml (w/v) CsCl and banded by centrifugation at 150,000 x g for 40 hours. The peak fractions corresponding to empty pseudocapsids (buoyant density 1.280 - 1.295 g/ml) and full particles (1.30-1.40) were collected, and pseudocapsids further purified and concentrated through a second sucrose cushion. Pseudocapsids were finally resuspended in buffer A at a protein concentration of 4 mg/ml.

*Formation of pCMVβ DNA-pseudocapsids complexes*

Affinity column purified pCMVβ (Clontech), containing the β-galactosidase gene under the control of the cytomegalovirus immediate early promoter, or pS65T (Clontech), containing the gene for green fluorescent protein (Chalfie *et al.*, 1994; with an enhanced fluorescence resulting from a mutation of serine 65 to threonine) under the control of the same promoter, was incubated for 15 minutes at room temperature with purified pseudocapsids, in a ratio of 1: 30 (w/w) in buffer A (150 mM NaCl, 10 mM Tris-HCl, pH 7.6, 10 $\mu$M CaCl$_2$, 5% glycerol, 0.01% Triton X-100). DNA and pseudocapsids controls were prepared similarly. Formation of DNA poly-L-lysine complexes was performed by incubating DNA-pseudocapsid complexes with poly-L-lysine (0.35 mg/ml) for 10 minutes, at room temperature.

*Electron microscopy pseudocapsid-DNA complexes*

Purified pseudocapsids (4 mg/ml) were incubated for 5 minutes on Formvar and carbon coated grids (200 mesh), the grids washed twice in double distilled water and then stained in a solution of 1 % phosphotungstic acid, pH 6, for 2 x 30 seconds. Grids were dried and observed on a JEOL JEM 1200EX electron microscope operating at 60 kV.

For visualisation of DNA and pseudocapsid-DNA complexes samples were prepared in 0.12 mg/ml cytochrome C, 0.5 M ammonium acetate, 1 mM EDTA, pH 7.5, at a DNA concentration of 1.75 µg/ml. Following spreading on water, the complexes were transferred to collodion coated copper grids (200 mesh) and stained with uranyl acetate (50 mM uranyl acetate, 50 mM HCl in 90% ethanol). Grids were rotary shadowed with Pt/Pd alloy and observed on a JEOL JEM 1200EX electron microscope operating at 60 kV.

### Transduction of marker gene encoding green fluorescent protein, in vitro

Monkey Cos 7 cells ($2 \times 10^4$), constitutively expressing the SV40 large T antigen, plated at half confluence on glass cover slips, were washed in DMEM lacking foetal calf serum (FCS) and incubated with pS65T DNA (Clontech) and pseudocapsid mixtures in DMEM lacking FCS (250 µl) with rocking every 15 minutes for 90 minutes. Excess DMEM, supplemented with 10% FCS, was then added to the cultures and incubation continued for 36 hours. The cells on coverslips were then washed in PBS and observed by fluorescence microscopy. Cells fluorescing green were counted on each coverslip.

For treatment with neuraminidase, cells were cultured in 200 mU neuraminidase during the incubation with DNA/pseudocapsid/poly-L-lysine mixtures, and then with 50 mU neuraminidase for the remainder of the incubation. Low pH experiments were carried out by culturing cells with DNA/pseudocapsid/poly-L-lysine mixtures in DMEM adjusted to pH 6.

### Transduction of marker gene, ß-galactosidase, in vivo

Six week old female nude (nu/nu) mice were injected subcutaneously at the back of the neck with DNA-pseudocapsid complex (5 µg DNA: 150 µg pseudocapsids), DNA (5 µg), or pseudocapsids (150 µg), alone. Animals were killed by cervical dislocation 1 or 6 weeks post injection and major organs snap frozen and stored at -70°C. One quarter of each organ was washed in phosphate buffered saline (PBS) incubated in fix (1 % formaldehyde, 0.2 % gluteraldehyde, 2 mM $MgCl_2$, 5 mM EGTA, 0.02% Nonidet P40 in PBS), washed and incubated overnight at room temperature in X-gal solution (3 mM $K_4Fe(CN)_6$, 3 mM $K_3Fe(CN)_6$, 1 mM $MgCl_2$, 0.05% 5-bromo-4-chlor-3-indoly-$\beta$-D-galactopyranoside in PBS). Enzymatic activity, detected by deposition of blue product, was determined by eye or low power light microscopy.

## RESULTS

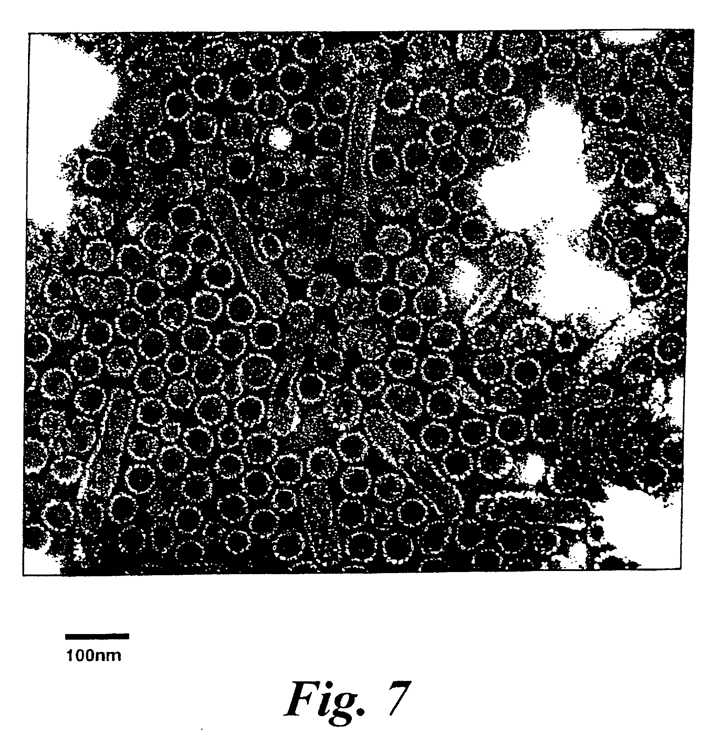

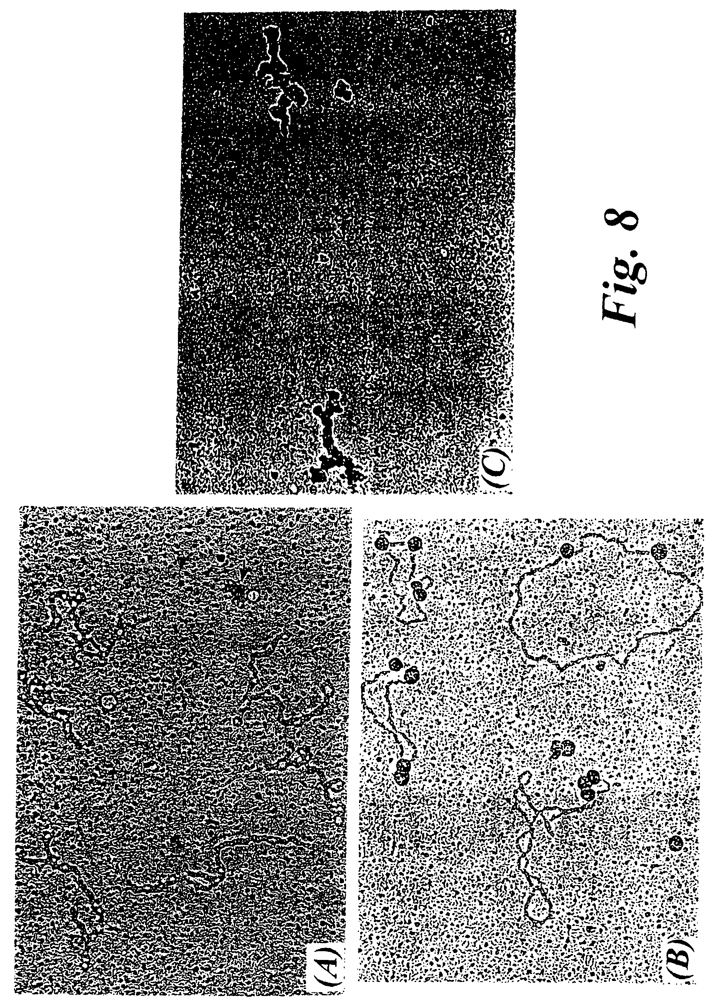

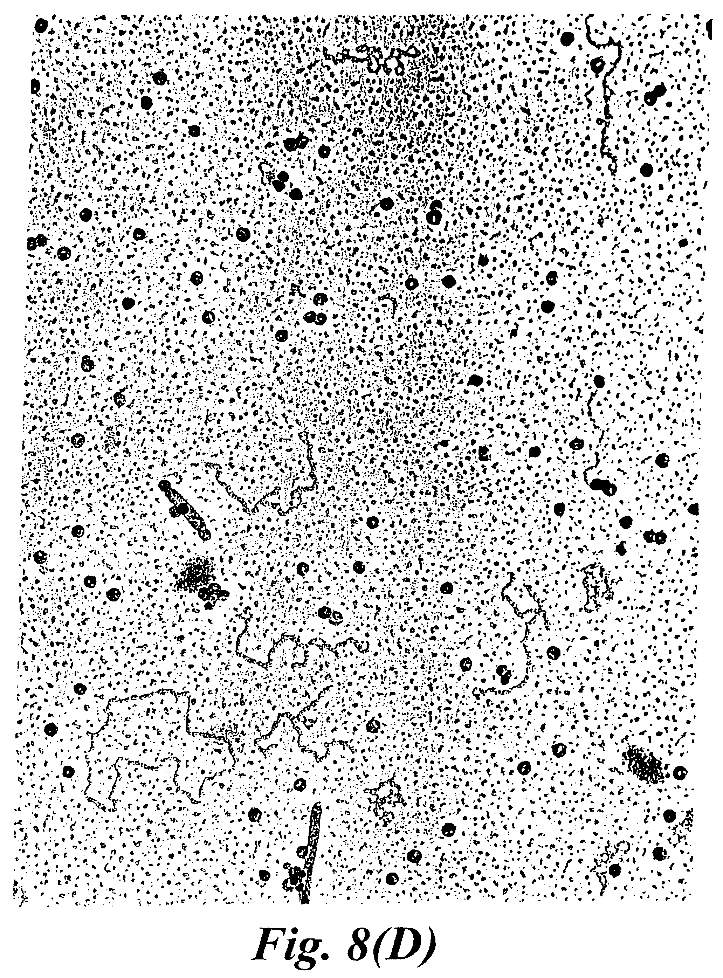

Purified pseudocapsid preparations, composed of greater than 99% VP1 (data not shown), were found to be assembled into icosahedral and tubular structures, as shown in the electronmicrograph (Figure 7). The majority of these structures were empty as determined by the lack of electron dense material within each form. Incubation of pseudocapsids with supercoiled DNA resulted in the formation of stable complexes. Observation by electromicroscopy (Figure 8), revealed that the 7.2 kbp supercoiled DNA (incubated alone, Panel A) associated with 2-4 pseudocapsids per DNA molecule and that the supercoil was relaxed by the association (Panel B). Further, comparison of measurements of supercoiled and complexed DNA molecules revealed an average shortening, indicating that 70% of the DNA was packaged by pseudocapsids. Condensation of the remaining free DNA could be achieved by adding poly-L-lysine to DNA-pseudocapsid complexes (Panel C), resulting in high affinity complexes of 200-300 nm in size. Incubation of full pseudocapsids (pseudocapsids with DNA packaged from the host cell; Pawlita *et al.*, 1996) with supercoiled DNA, result in less than 5% complex formation (Panel D), indicating that only empty pseudocapsids can package exogenously added DNA.

To test the ability of the different complexes to transfer DNA to cells, plasmid encoding the gene for green fluorescence protein (GFP; Chalfie *et al.*, 1994) was used as a marker. The results are shown in Figure 9A. Cells incubated in DNA alone (DNA), or DNA-full pseudocapsid mixtures (DNA+full p'caps) did not take up sufficient DNA to express detectable GFP. However, 0.5% of cells incubated with DNA-pseudocapsid complexes (DNA+p'caps) expressed fluorescing protein after 36 hours in culture. The number of positive cells increased to more than 4 % if complexes were further condensed by the addition of poly-L-lysine (DNA+p'caps+PLK), which was not due to the formation of DNA-poly-L-lysine complexes alone, as less than 0.05% of cells incubated with these preparations expressed GFP (DNA+PLK).

The transduction of DNA by DNA-pseudocapsid-poly-L-lysine complexes, however, could be demonstrated to have different properties to natural polyoma virions, or DNA-pseudocapsid complexes, as shown in Figure 9B. Neuraminidase removes surface sialic acid residues essential for initial interaction of polyoma virus with cells, and viral infectivity. Treating cell cultures with neuraminidase (+neuraminidase) during and after incubation with DNA-pseudocapsid complexes, inhibited any detectable transfer of the GFP gene (closed bars). However, similar treatment of cells incubated with DNA-pseudocapsid-poly-L-lysine complexes (open bars) reduced the number of transduced cells by only 50-60%, compared to controls. Further, incubating complexes on the cells in medium at pH 6 (Low pH), which enhances natural virion infectivity (Fogel and Sachs, 1959), increased the DNA-pseudocapsid uptake 6-fold, but had no significant effect on DNA-pseudocapsid-poly-L-lysine uptake. These results indicate that DNA-pseudocapsid complexes are taken up into cells in a manner similar to natural virions, whereas DNA-pseudocapsid-poly-L-lysine complexes are not.

Based on the *in vitro* data, *in vivo* experiments were carried out with DNA-pseudocapsid complexes. The marker gene selected for these experiments encoded the β-galactosidase gene under the control of the human CMV immediate early promoter (pCMVβ, Clontech). Preparations were introduced into athymic nude (nu/nu) mice subcutaneously, being the same route used to introduce natural polyoma virus for tumour formation studies (Eddy, 1969). Following inoculation, mice were killed at 1 and 6 weeks, and organs tested for bacterial β-galactosidase activity using X-gal as substrate, which results in deposition of blue product in the presence of the enzyme. The results of staining of a selection of major organs are shown in Table 1A.

Table 1A

| Expression of the marker gene, β-galactosidase, in organs of athymic nude mice | | | | |
|---|---|---|---|---|
| **SHORT TERM** | | | | |
| **Tissue/Mouse** | **DNA 1** | **PART 1** | **D + P 1** | **D+ P 2** |
| **Liver** | -ve | -ve | -ve | + |
| **Kidney** | -ve | -ve | + | -ve |
| **Brain** | -ve | -ve | -ve | -ve |
| **Skin (Bk)** | -ve | -ve | -ve | -ve |
| **Skin (N)** | +++ | -ve | + | + + |
| **LONG TERM** | | | | |
| **Tissue/Mouse** | **DNA 2** | **PART 2** | **D+P 3** | **D+P 4** |
| **Liver** | -ve | -ve | -ve | + |
| **Kidney** | -ve | -ve | -ve | -ve |
| **Spleen** | -ve | -ve | -ve | -ve |
| **Brain** | -ve | -ve | + + | + + + + |
| **Skin (Bk)** | -ve | -ve | -ve | -ve |
| **Skin (N)** | -ve | -ve | + + | + + + |

Eight animals were treated, two each with DNA (DNA 1 and 2), or pseudocapsids (PART 1 and 2), alone and four with DNA-pseudocapsid preparations (D+P1-4). After one week (short term) skin at the site of inoculation (N) was found to express β-galactosidase activity in the mice injected with DNA alone, or with DNA-pseudocapsid complexes. A low level of signal was found also in liver and kidney samples of DNA-pseudocapsid injected mice. However, after six weeks, no expression of β-galactosidase could be detected in mice injected with DNA alone, but skin sites were still positive in the mice inoculated with DNA-pseudocapsid complexes. Further, unexpectedly, highest expression of β-galactosidase was found in brain tissue of these mice. The stained brain tissues are shown in Figure 10, panel A, where it can be seen that neither DNA (DNA) or pseudocapsids (CAPSID) alone resulted in any blue colouration of the tissue when incubated in X-gal, whereas, the brain of D+P4 (D+C) mouse is extensively stained. The expression is localised to discrete areas in the brain, as shown at a higher magnification. In Figure 10, panel B. The brain of the second DNA-pseudocapsid injected mouse (D + P3) was also significantly more positive than any other tissues tested, although the intensity of staining was not as great as for the D+P4 mouse (data not shown).

The influence of the route of administration of the pseudocapsids is shown in Table 2A.

**Table 2A** Summary of β-galactosidase expression in tissue

| | 1 WEEK | | | 7 WEEK | |
|---|---|---|---|---|---|
| **High level expression** | | **Spleen** | | | **Heart** |
| **Intermediate expression** | Spleen | Kidney<br>Heart | | | Spleen<br>Kidney<br>Brain |
| **Low or not expression** | Heart<br>Kidney<br>Lung<br>Liver<br>Parotid<br>Skin<br>Mammary Gland<br>Brain | Lung<br>Liver<br>parotid<br>Skin<br>Mammary Gland<br>Brain | | Spleen<br>Heart<br>Kidney<br>Lung<br>Liver<br>Parotid<br>Skin<br>Mammary Gland<br>Brain | Lung<br>Liver<br>Parotid<br>Skin<br>Mammary Gland |

| BRAIN | | | | | | | |
|---|---|---|---|---|---|---|---|
| | **DNA** | | | | **D + C** | | |
| **TIME/ROUTE** | SC | IV | IP | IN | SC | IV | IP | IN |
| **Short term** | + | -- | -- | -- | + | -- | -- | -- |
| **Long term** | -- | -- | -- | -- | + + | + | + + + | + + |

| | | |
|---|---|---|
| DNA | = | 5μg DNA dose |
| D + C | = | Pseudocaps |
| SC | = | Subcutaneous |
| IV | = | Intravaneous |
| IP | = | Intraperitoneal |
| IN | = | Intranasal |

EP 0 977 596 B1

To confirm that the staining observed was due to the introduced DNA and not the endogenous gene, a polymerase chain reaction (PCR) was carried out with primers selected from within the CMV promoter and the bacterial gene. This technique also allowed semi-quantitative assessment of gene levels.

## PCR

Denatured DNA (500ng), prepared using the QIAamp Tissue kit (QIAGEN), was added to a PCR reaction containing 100pM primers (GTCAGATCGCCTGGAGACGCCAT and TTCCAGATAACTG CCGTCACTCC, nucleotides 548-570 and 1538-1516 of pCMVβ) (Stratagene, Cambridge, UK) and amplified for 35 cycles. Parallel reactions were carried out with samples to which $10^2$ copies of plasmid DNA had been added to control for possible enzyme inhibition. Products, separated by agarose gel electrophoresis, were transferred by Southern blotting to nylon membrane (Biodyne B, Pall, Portsmouth, Hampshire) and probed with $^{32}$P-labelled pCMVβ. The resulting autoradiograph was digitised (Nikon Scantouch 10, Tokyo, Japan) and band intensities measured (Kodak Digital Science 1D program, Kodak Scientific Imaging Systems, Rochester. NY, USA). Copy number was determined by comparing the intensity of the bands with products from similar PCRs of plasmid DNA mixed with aliquots of DNA extracted from untreated nude mouse tissues.

PCR performed on DNA isolated from brain samples taken 7 weeks following treatment with pseudocapsid-DNA complexes, resulted in amplification of products of the expected size (990 bp; Figure 17A, panels a and b). These products hybridised specifically to radiolabelled pCMVβ (tracks labelled D+P), confirming the identity of the introduced β-galactosidase gene. PCR products were not detected after 7 weeks at significant levels in brains of animals treated with DNA only (tracks labelled DNA). Notably, levels of PCR products in the brain samples differed, depending on the route of administration, that is, i.p., i.n. and s.c. delivery were markedly better at delivering DNA to brain than the i.v. route.

To analyse this effect further, the levels of marker gene were compared in brain and heart. PCR data were quantified by densitometry and demonstrated a correlation between bacterial β-galactosidase activity and levels of DNA detected by PCR in the brain (Figure 17B, black bars), supporting the notion of route-dependent variation. Where DNA was introduced alone, little or no gene transfer was detected by any route (white bars). In contrast, in heart tissue, enzymatic and PCR assays at 7 weeks demonstrated expression of β-galactosidase and the presence of the gene in mice treated with DNA only, as well as with pseudocapsid-DNA complexes (Figure 17C). However, here there was less correlation between gene expression and levels of PCR products and, unlike in brain, there was no distinct pattern associated with route of administration. I.v. administration did, however, result in high levels of detected DNA when DNA was administered alone, and high levels of gene expression in the case of DNA-pseudocapsid complexes. This observation may be explained by high concentrations of DNA or DNA-pseudocapsid complexes rapidly reaching the heart *via* the blood stream by this delivery route.

In three separate experiments, using a total of 35 mice, a general pattern has emerged, as summarised in Table 2A (where data from all routes of administration have been combined). That is, after 1 week, transient expression of β-galactosidase was detected in heart, spleen and kidney for both naked DNA and DNA-pseudocapsid complexes, by all routes of administration, with levels being consistently higher when pseudocapsids were used. This difference was greatly enhanced with time and, by 7 weeks, most expression from mice treated with DNA alone had been lost, whereas expression was enhanced and sustained with DNA-pseudocapsid delivery. In addition, in the latter, expression in the brains of the animals was observed.

Introduction of pseudocapsids was completely asymptomatic in these studies, or in other experiments when injected into the third ventricle of the brain in immunocompetent rats (see below). The observed distribution of expression among organs may be a reflection of the activity of the CMV promoter, which has been reported to vary in different tissues *in vivo*[21]. However, our data suggest that pseudocapsids themselves may influence expression patterns, and recent collaborative work elsewhere (T. Dalianis, personal communication) shows that other recombinant plasmid DNA constructions derived from the mouse polyoma virus genome, can also be detected by PCR in brain when introduced in complexes with pseudocapsids. *In vitro,* DNA introduced with pseudocapsids has been shown to integrate into the host chromosome[22]. *In vivo,* pseudocapsid mediated gene transfer also appears to be stable. Ongoing experiments show that expression of input DNA can still be identified at least 3.5 months later; longer term experiments are in progress.

I.v. administration, which resulted in high levels of gene delivery in the heart, was the least successful route to the brain. Administration intranasally on the other hand, was effective in delivering DNA to this site. Thus transmission of pseudocapsids to the CNS may not be proceeding *via* the blood brain barrier, but by an alternative route, for instance by retrograde axonal transport, in a manner similar to that seen with herpes simplex virus[23,24]. Mouse polyoma virus

[21] Baskar, J.F., *et al.* The enhancer domain of human cytomegalovirus major immediate-early promoter determines cell type specific expression in transgenic mice. *J. Virol*; **70**: 3207-3214 (1996).
[22] Forstova, J., *et al.* Polyoma virus pseudocapsids as efficient carriers of heterologous DNA into mammalian cells. *Hum Gene Ther;* **6**: 297-306 (1995).

may not previously have been detected in the CNS due to inefficient replication at this site, since most assays for the virus (for example, plaque assays) depend upon this property for generating sufficient materials for measurement.

The present new approach for *in vivo* delivery of genes to the CNS provides considerable advantages over previously used viral and nonspecific methods, which have drawbacks with regard to safety and efficiency[25,26,27]. Viral vectors have shown promise in terms of levels and persistence of expression, but carry viral DNA and a risk of reversion in attenuated strains, and non-viral vectors do not result in long-term expression [28,29,30]. The pseudocapsids of the invention thus offer an alternative carrier approach as they mimic specific viral mediated uptake, but do not in themselves contain viral genetic information, nor do they require helper virus functions. Access to the CNS is restricted by the blood brain barrier, necessitating administration of DNA, in many cases, by intracerebral injection[31]. Peripheral administration of viruses, such as the neurotrophic HSV and adenovirus, gives variable results[32,33,34], and an inflammatory response to these viruses may also limit their use[35,36]. In this study, pseudocapsid mediated DNA delivery resulted in consistent and sustained expression in neuronal tissue following peripheral administration (including the non-invasive intranasal route) with low, or no, toxicity. These results suggest that the pseudocapsid approach could form the basis of future gene therapy protocols, particularly for the nervous system.

**Example 13(ii):** *Expression of ß-galactosidase gene in rat brain following stereotactic administration of DNA and DNA/pseudocapsid complexes*

Plasmid DNA (0.5μg; pCMVβ), encoding the β-galactosidase gene, complexed to pseudocapsids (at a ratio of 1:30 w/w), or alone, was introduced by stereotactic injection into the third ventricle of rat brains. Animals were killed at 1 and 7 weeks and tested for antibody response to pseudocapsids and expression of β-galactosidase.

1 week following injection, high level expression of β-galactosidase was detected by X-gal staining in the cells lining the third ventricle in animals treated with both DNA and DNA/pseudocapsid complexes. Further expression was detected in the needle tract for DNA/pseudocapsid treated rats and in small patches of cells distant from the point of injection. After 7 weeks, expression was also detected in the cerebellum.

There were no signs of adverse effects of pseudocapsid treatment in the animals as determined by their feeding patterns, weight changes or other behaviour. However, there was a significant immune response to VP1 in animals treated with pseudocapsids.

**Example 13(iii):** *Pseudocapsid mediated expression of ß-galactosidase in immunocompetent mice*

6 week old female balb c mice were injected intraperitoneally, or treated intranasally with either DNA (5μg; pCMVβ) alone, or DNA complexed with pseudocapsids. Mice were killed at I and 7 weeks and tested for antibody production and expression of β-galactosidase by X-gal staining.

1 week following injection, expression of β-galactosidase was detected in heart, liver, kidney and spleen in mice injected intraperitoneally with DNA or DNA/pseudocapsid complexes. In mice injected with DNA/pseudocapsid complexes, expression was additionally found in brain. In animals treated with complexes intranasally, a low level of expression was detected only in kidney at 1 week. At 7 weeks, no expression was detected in tissues from mice treated with DNA alone, whereas high levels of expression were found in brain, kidney and heart, and moderate levels in liver and spleen for mice treated with DNA/pseudocapsid complexes both intraperitoneally and intranasally.

Sustained expression of the marker gene was observed despite a significant antibody titre against VP1 found in mice at 1 week following treatment with pseudocapsid/DNA complexes. The titre was lower in mice treated intranasally and diminished with time.

[23] Sun, N., Cassell, M.D. and Perlman, S. Anterograde, transneuronal transport of herpes simplex virus type 1 strain H129 in the murine visual system. *J Virol; 70*: 5405-5413 (1996).
[24] Engel, J.P., Madigan, T.C. and Peterson, G.M. The transneuronal spread phenotype of herpes simplex virus type I infection of the mouse hind footpad. *J Virol;* **71**: 2425-35 (1997).
[25] Karpati, G., Lochmuller, H., Nalbantoglu. J. and Durham, H. The principles of gene therapy for the nervous system. *Trents Neurosci;* **19**: 49-54 (1996).
[26] Blomer, U., Naldine, L., Verma, I.M., Trono D. and Gage, F.H. Applications of gene therapy to the CNS. *Hum Mol Genet;* **5**: 1397-1404 (1996).
[27] Ho, D.Y. and Sapolsky, R.M. Gene therapy for the nervous system. *Sci Am*; **276**: 80-85 (1997).
[28] Verma, I.G. and Somia, N. Gene therapy - promises, problems and prospects. *Nature;* **389**: 238-242 (1997).
[29] Freidmann, T. Overcoming the obstacles to gene therapy. *Sci Am;* **276**: 80-85 (1997).
[30] Felgner, P. L. Nonviral strategies for gene therapy. *Sci Am;* **276**: 86090 (1997).
[31] Karpati, G., Lochmuller, H., Nalbantoglu, J. and Durham, H. The principles of gene therapy for the nervous system. *Trends Neurosci;* **19**: 49-54 (1996).
[32] Ghadge, G.D., *et al.* DNS gene delivery by retrograde transport of recombinant replication-defective adenorviruses. *Gene Ther;* **2**: 132-7 91995).
[33] Draghia, R., *et al.* Gene delivery into the central nervous system by nasal instillation in rats. *Gene Ther;* **2**: 418-23 (1995).
[34] Huard, J., *et al.* The route of administration is a major determinant of the transduction efficiency of rat tissues by adenoviral recombinants. *Gene Ther;* **2**: 107-15 (1995).
[35] Wood, M.J., Byrnes, A.P., Rabkin, S.D., Pfaff, D.W. and Charlton, H.M. Immunological consequences of HSV-1-mediated gene transfer into the CNS. *Gene Ther* (1994).
[36] Wood, M.J., Charlton, H.M., Wood, K.J., Kajiwara, K. and Byrnes. A.P. Immune responses to adenovirus vectors in the nervous system. *Trends Neurosci;* **19**: 497-501 (1996).

**Example 14: *Long-term expression of the marker gene, ß-galactosidase, in ex vivo rabbit cornea cultures***

**METHODS**

***Preparation of pseudocapsids, DNA and complexes***

as for Example 13.

***Transduction of marker gene to corneas ex vivo***

Corneas were harvested from outbred female New Zealand White rabbits, divided into quarters and incubated with DNA-pseudocapsid complex (1 μg DNA: 30 μg pseudocapsids), DNA (1 μg) alone, or as a positive control, 1.5 x $10^7$ pfu recombinant adenovirus containing the β-galactosidase gene under the control of the human cytomegalovirus immediate early promotor (RAd35: Wilkinson and Ackrigg, 1992). Cultures were maintained for 1 or 4 weeks in minimal essential medium (MEM; Gibco-BRL) supplemented with 2 % foetal calf serum (FCS) and then corneas were fixed and stained for β-galactosidase activity, as described for Example 13.

**RESULTS**

The ability of pseudocapsids to specifically transfer DNA to differentiated tissues in organ culture was tested. Rabbit corneas were selected for this experiment as they may be cultured *ex vivo* for up to 4 weeks following dissection and, in culture, have both dividing (epithelial) and non-dividing (endothelial) cell layers. In addition, the endothelial cells are neural in origin (Tuft and Coster, 1990). Corneas were incubated with DNA-pseudocapsid complexes, DNA alone, or recombinant adenovirus encoding the β-galactosidase gene and analysed at two time points for expression of β-galactosidase by staining with X-gal. Only corneas incubated with recombinant adenovirus were found to express β-galactosidase after 1 week in culture. However, after 4 weeks expression of β-galactosidase was found in the endothelial cell layer of corneas incubated with DNA-pseudocapsid complexes (Figure 11, panel A). Significant numbers of dividing epithelial cells, sloughed off from the culture and growing on the surface of the culture dish, were also positive for the marker protein (data not given). No staining was observed in endothelial cell layers in corneas incubated in DNA alone (panel B), but expression was maintained over the period of culture in cells infected with the recombinant adenovirus (panel C), at a level similar to that achieved with DNA-pseudocapsid complexes.

**INTRODUCING GENES TO SUPPLEMENT MISSING OR REDUCED FACTORS**

**Example 15: *Increasing levels of dopamine by over-expression of tyrosine hydroxylase.***

Humans tyrosine hydroxylase cDNA under the control of constitutive viral (broad activity), or its own (neuronal specific) promoter was packaged into pseudocapsids by the standard protocol described above.

***In Vivo***

Pseudocapsid complexes are then introduced by sub-cutaneous, intraperitoneal, intravenous injection, or intranasal administration to a mammal. Alternatively, complexes are injected by stereotactic injection directly into the striatum of the brain.
For systemic introduction, if considered necessary, temporary immunosuppression may be carried out for up to one week following introduction of the pseudocapsid complexes.

***In Vitro***

For *in vitro* administration, foetal neurons, neuronal multipotent cell lines, adrenal chromaffin cell, glial cells, myoblasts or fibroblasts are incubated with pseudocapsid complexes for 1 hour in serum free medium and cultured overnight in supplemented growth medium. Transfected cells are then injected into the appropriate site of the nervous system, where therapy is required.
In a varient method an anti apoptotic gene is used eg. neural apoptosis-inhibitory protein (Roy *et al* 1995 Cell, 80;167).
The above method can be used for achieving expression of any neuropeptide from its cDNA, or, for instance, introduction of antisense to prion proteins.
Other genetic material of interest for the treatment of neurological diseases is given in the following table:

Examples of therapeutically useful direct experimental gene transfer into cells of the nervous system *in vitro* and *in vivo* (animals), from Karpati *et al* (1996) Trends Neurosic: **19, 2, 49-54.**

| Gene | Cell or tissues | Relevant diseases | Ref. |
|---|---|---|---|
| Nerve growth factor | PC12 cells or sympathetic neurons | Alzheimer's disease Dysautonomia | 66 |
| Tyrosine hydroxylase | Cultured striated neurons | Parkinson's disease | 65 |
| Antisense construct for astrocyte glial librillary-acidle protein | Cultured astrocytes | Gliosis | 68 |
| Nerve growth factor | Septo-hippocampal cell culture | Alzheimer's disease | 26 |
| PMP22 | Cultured rat Schwann cells | Tomaculous neuropathy | 58 |
| Tyrosine hydroxylase | Partially denervated rat striatum | Parkinson's disease | 64 |
| Nerve growth factor | Denervated superior cervical ganglion of rats | Dysautonomia | 67 |
| Hypoxanthine-guanine phosphoribosyl transferase | Unspecified areas of mouse brain | Lesch-Nyhan syndrome | 60 |
| Tyrosine hydroxylase | Denervated rat striatum | Parkinson's disease | 65 |

26   Yang, K. *et al.* (1994) *Neurosci. Lett.* 182, 291-294
58   Snipes, G.J. and Suter, U. (1995) *Brain Pathol. 5, 233-247*
60   Pallela, T.D. *et al. (1989) Gene 80, 137-144*
64   During, M.J. *et al* (1994) *Science* 266, 1399-1403
65   Horellou, P. *et al* (1994) *NeuroReport* 6, 49-53
66   Geschwind, M.D. *et al* (1994). *Mol. Brain Res.* 24, 327-335
67   Federoff, H.J. *et al* (1992) *Proc. Natl. Acad. Sci. USA* 89, 1636-1640

EP 0 977 596 B1

*Cancer Treatment by Introducing of Drug Sensitivity Genes*

**Example 16:** *Inducing drug sensitivity to Gancyclovir by introduction of herpes simpler virus thymidine kinase (also suitable for cytosine deaminase)*

Genes for the appropriate sensitisation regime are packaged into pseudocapsids by the standard protocol and introduced by injection directly into the tumour mass. Two days later gancyclovir is administered systemically.

*Introducing Peptide Nucleic Acids (PNA)/peptides/proteins*

**Example 17:** *Introduction of PNA to produce an antisense agent against prion protein*

Up to 18mer PNAs (Buchardt *et al* 1993 Trends Biotech, 11; 384) are synthesised as antisense sequences to the human prion protein gene and then covalently linked to the non-coding region of mouse polyoma virus (5021-173 bp through the origin of replication). Alternatively linkage is made to shorter DNA sequences from this region, to assist in packaging. Modified PNAs are then interacted with pseudocapsids at a ratio of 1:30 (w/w) for 15 minutes at room temperature.

Alternatively, pseudocapsids are disassembled in the presence of 10 mM EGTA and 100 mM dithiothreitol, mixed with PNAs at a 30:1 (w/w) ratio and dialysed against buffer (150 mM NaCl, 10 mM Tris-HCl pH 7.4, 0.01 microM $CaCl_2$, 0.05% Triton X 100, 5 % glycerol) overnight, at 4 degC. Reassembled pseudocapsid-PNA complexes are then concentrated by ultrafiltration and administered to a mammal subcutaneously, interperitoneally, intravenously by injection, or intranasally. Alternatively, complexes are injected by stereotactic injection directly into the brain.

Neuroactive peptides or proteins are either packaged by incubation with dis-assembled pseudocapsids, or covalently linked to DNA and then packaged, as above. Alternatively, peptides or proteins are covalently linked directly to pseudocapsids by transglutamination, and then introduced, as above.

For systemic introduction, if considered necessary, temporary immunosuppression may be carried out for up to one week following introduction of the pseudocapsid complexes.

For *in vitro* administration, foetal neurons, neuronal multipotent cell lines, adrenal chromaffin cell, glial cells, myoblasts or fibroblasts are incubated with pseudocapsid complexes for 1 hour in serum free medium and cultured overnight in supplemented growth medium. Transfected cells are then injected into the appropriate site of the nervous system, where therapy is required.

Although a skilled person will be able to determine an appropriate dosage for a mammal of interest, for administration to an adult human being a dosage of approximately 10 milligrams of DNA to 300 milligrams of pseudocapsids should be appropriate.

## DISCUSSIONS

These results demonstrate that the pseudocapsid gene transfer system is able to mediate transfer of marker plasmids of at least 7.2 kbp in size into cells *in vitro* and *in vivo. In vivo* pseudocapsids exhibited unexpected tissue tropism in that, when introduced subcutaneously, one of the major target organs is the central nervous system. That pseudocapsids can transfer DNA to non-dividing cells of neural origin is further supported by the high level expression of marker plasmid, demonstrated in corneal endothelium in *ex vivo* cultures.

Tracing the tissue tropism of polyoma virus by tumour formation has shown that no tumours form in brains of mice injected subcutaneously with virus, although tumours are induced at many other sites throughout the animal (Eddy, 1969). Further, detection by polymerase chain reaction has not revealed presence of viral DNA in brains of immunocompromised mice infected with the virus. Hence, the tissue tropism property of the pseudocapsids of the invention is quite unexpected.

## REFERENCES

ANDERSON, W.F. Human gene therapy. (1992). *Science* **256**, 808-813.

BARR, S.M., KECK, K. & APOSHIAN, H.V. (1979). Cell-free assembly of a polyoma-like particle from empty capsid and DNA. *Virology* **96**, 656-659.

CHALFIE, M., TU. Y., EUSKIRCHEN, G., WARD, W.W. & PRASHER, D. Green fluorecent protein as a marker for gene expression. Science 1994; 263: 802-805.

COLLETT, M.S. & ERIKSON, R.L. (1978). Protein kinase activity associated with the avian sarcoma virus *src* gene product. *Proc. Natl. Acad. Sci. USA* **75**, 2021-2024.

COURTNEIDGE, S.A. & SMITH, A.E. (1983). Polyoma virus transforming protein associates with the product of the c-src cellular gene. *Nature* **303**, 435-439.

DILWORTH, S.M. (1982). Protein kinase activities associated with distinct antigenic forms of polyoma virus middle T-antigen. *EMBO J.* 1, 1319-1328.

DILWORTH, S.M. & GRIFFIN, B.E. (1982). Monoclonal antibodies against polyoma virus tumor antigens. *Proc. Natl. Acad. Sci.* U.S.A. 79, 1059-1063.

EDDY, B.E. (1969). In, *Virology Monographs* (1969). **7**, Springer-Verlag (Vienna, New York) pp. 1-112.

FOGEL, M, & SACHS. L. The *in vitro* and *in vivo* analysis of mammalian tumour viruses. II The haemagglutinating system of the polyoma virus. Brit. J. Cancer 1959; 13: 266-281.

FORSTOVÁ, J., KRAUZEWICZ, N., WALLACE, S., STREET, A.J., DILWORTH, S.M., BEARD, S. & GRIFFIN, B.E. (1993). Cooperation of structural proteins during late events in the life cycle of polyomavirus. *J Virol.* **67**, 1405-1413.

GRAHAM, F.L. & VAN DER EB, A. (1973). Transformation of rat cells by DNA of human adenovirus 5. *Virology* **54**, 536-539.

GRIFFIN, B.E. & MADDOCK, C. (1979). New classes of viable deletion mutants in the early region of polyoma virus. *J. Virol.* **31**, 645-656.

HOEIJMAKERS, J.H.J., ODIJK, H. & WESTERVELD, A. (1987). Differences between rodent and human cell lines in the amount of integrated DNA after transfection. *Exp. Cell Res.* **169**, 111-119.

KOCH, I., HOFSCHNEIDER, P.H., LOTTSPEICH, F., ECKERSKORN, C. & KOSHY, R. (1990). Tumour-related expression of a translation-elongation factor like protein. *Oncogene* **5**, 839-843.

LAEMMLI, U.K. (1970). Cleavage of structural proteins during the assembly of the head of bacteriophage T4. *Nature* **227**, 680-685.

MAYNE, L.V., JONES, T., DEAN, S.W., HARCOURT, S.A., LOWE, J.E., PRIESTLEY, A., STEINGRIMSDOTTIR, H., SYKES, H., GREEN, M.H.L. & LEHMANN, A.R. (1988). SV40-transformed normal and DNA-repair-deficient human fibroblasts can be transfected with high frequency but retain only limited amounts of integrated DNA. *Gene* **66**, 65-76.

MONTROSS, L., WATKINS, S., MORELAND, R.B., MAMON, H., CASPAR, D.L.D. & GARCEA, R. (1991). Nuclear assembly of polyomavirus capsids in insect cells expressing the major capsid protein VP1.*J. Vi rol.* **65**, 4991-4998.

MORGAN, R.A. & ANDERSON. W.F (1993). *Ann. Rev. Biochem.* **62**, 191-217.

MULLIGAN, R.C. (1993). The basic science of gene therapy. *Science* **260**, 926-932 (1993).

PAWLITA, M., MULLER, M., OPPENLANDER, M., ZENTGRAF, H. & HERRMANNM, M. DNA encapsidation by viruslike particles assembled in insect cells from the major capsid protein of B-lymphotrophic papovavirus. J. Virol. 1996; 70: 7517-7526.

PONTÉN, J. (1971). *Virology monographs* **8**, pp. 46-92 (Springer Verlag, Wien).

SLILATY, S.N. and APOSHIAN, H.V. (1983). Science, 220, 725-727.

SLILATY, S.N., BERNS, K.I. and APOSHIAN, H.V. (1982) J. Biol. Chem. 257: 6671-6675.

SMITH, A.E., SMITH, R., GRIFFIN, B. & FRIED, M. (1979). Protein kinase activity associated with polyoma virus middle T antigen *in vitro. Cell.* **18**, 915-924.

STRAUSS, M.M., STREULI, C.H. & GRIFFIN, B.E. Efficient oligodeoxyribonucleotide-directed deletion mutagenesis using pEMBL vectors: removal of early region introns from polyoma virus mutants. *Gene* **49**, 331-340 (1986).

STRAUSS, M., HERING, S., LUBBE, L. & GRIFFIN, B.E. (1990). Immortalisation and transformation of human fibroblasts by regulated expression of polyoma virus T antigens. *Oncogene* **5**, 1223-1229.

SOEDA, E., ARRAND, J.R., SMOLAR, N., WALSH, J.E. & GRIFFIN, B.E. (1980). Coding potential and regulatory signals of the polyoma virus genome. *Nature* **283**, 445-453. TUFT, S.J. & COSTER, D.J. The corneal endothelium. Eye 1990; 4: 389-424

TÜRLER, H & BEARD, P. (1985). Simian virus 40 and polyoma virus: growth, titration, transformation and purification of viral components. In *"Virology: A practical approach"* (ed. Mahy, B.W.J.) pp. 169-192 (IRL press, Oxford).

WAGNER, E., ZATLOUKAL, K., COTTEN, M., KIRLAPPOS, H., MECHTLER, K., CURIEL, D.T. & BIRNSTIEL, M.L. (1992). Coupling of adenovirus to transferrin-polylysin/DNA complexes greatly enhances receptor-mediated gene delivery and expression of transfected genes. *Proc. Natl. Acad. Sci.* U.S.A. **89**, 6099-103.

WILKINSON, G.W.G. & AKRIGG. A. Consitutive and enhanced expression from the CMV major IE promoter in a defective adenovirus vector. Nucleic Acids Res 1992; 20: 2233

## Claims

1. Use of a pseudocapsid formed from a papovavirus major capsid antigen and excluding minor capsid antigens, which pseudocapsid has exogenous material associated therewith, in the manufacture of a pseudocapsid composition for treating a neuronal host cell so that the exogenous material is taken up by the cell and is biologically functional in that cell to produce a beneficial effect in the cell or a mammal containing the cell, for the treatment and/or prevention of a neurological disease; or for the detection of a neuronal cell, provided that the pseudocapsid is not one formed from at least one semi-purified or pure SV40 capsid protein and a constituent selected from the group consisting of an exogenous DNA encoding an exogenous protein or peptide product, or encoding a therapeutic RNA, or itself a therapeutic product, a vector comprising exogenous DNA encoding an exogenous protein or peptide product, or encoding a therapeutic RNA, or itself a therapeutic product, an exogenous RNA encoding an exogenous protein or peptide product or itself a therapeutic product, a vector comprising an exogenous RNA encoding an exogenous protein or peptide product, or itself a therapeutic product, an exogenous protein or peptide product, and antisense RNA, ribozyme RNA or any RNA or DNA which inhibits or prevents the expression of undesired protein/s in a mammalian cell, and optionally further comprising operatively linked regulatory elements sufficient for the expression and/or replication of said exogenous therapeutic RNA or of an exogenous protein or peptide in a mammalian cell.

2. Use of a pseudocapsid formed from a papovavirus major capsid antigen and excluding minor capsid antigens, which pseudocapsid has exogenous material associated therewith, in the manufacture of a pseudocapsid composition for treating a neuronal host cell so that the exogenous material is taken up by the cell and is biologically functional in that cell, to produce a beneficial effect in the cell or a mammal containing the cell, for the treatment and/or prevention of a neurological disease; or for the detection of a neuronal cell, wherein the neuronal host cell is derived from or present in the central nervous system of a mammal.

3. Use as claimed in Claim 1 or 2 wherein the neuronal host cell is derived from or present in the brain of a mammal.

4. Use as claimed in Claim I wherein the major capsid antigen is from one or more of human papilloma virus; mouse polyoma virus, bovine papilloma virus; or the human polyoma virus BK.

5. Use as claimed in Claim 2 wherein the major capsid antigen is from one or more of human papilloma virus; mouse polyoma virus, bovine papilloma virus; simian virus 40; or the human polyoma virus BK.

6. Use as claimed in Claim 1 or 2 wherein the major capsid antigen is VP1 from mouse polyoma virus.

7. Use as claimed in any previous claim wherein the neurological disease is selected from Alzheimers' or Parkinsons' disease.

8. Use as claimed in any one of Claims 1 to 7 wherein the pseudocapsid composition comprises a pharmacologically acceptable carrier.

9. Use as claimed in any one of Claims 1 to 8 wherein the exogenous material is genetic material.

10. Use of a pseudocapsid as claimed in Claim 9 wherein the genetic material comprises all or a coding part of at least one gene.

11. Use as claimed in Claim 10 wherein the gene is selected from one or more of nerve growth factor, tyrosine hydroxylase or neural apoptosis-inhibitory protein.

12. Use as claimed in Claim 10 or 11 wherein expression of the gene or genes is under the control of a nervous system-specific promoter or a promoter specific for a particular neuronal cell subpopulation.

13. Use as claimed in Claim 12 wherein the promoter is selected from the promoters for neurofilament light chain, neuron-specific enolase, tyrosine hydroxylase, dopamine β-hydroxylase, glial acidic-fibrillary protein, Tα1 -tubulin, β-chain of platelet derived growth factor, myelin basic protein, and preproenkephalin.

14. Use of a pseudocapsid as claimed in any one of Claims 1 to 8 wherein the exogenous material is a protein or a polypeptide.

15. Use of a pseudocapsid as claimed in any one of Claims 1 to 8 wherein the protein or polypeptide is an antibody or a fragment thereof.

16. Use of a pseudocapsid as claimed in any one of Claims 1 to 8 wherein the exogenous material is a pharmacologically active compound.

17. Use of a pseudocapsid as claimed in any one of Claims 1 to 8 wherein the exogenous material is a peptide nucleic acid.

18. Use of a pseudocapsid as claimed in any one of Claims 1 to 8 wherein the exogenous material comprises a complex of biological macromolecules.

19. Use of a pseudocapsid as claimed in Claim 18 wherein the complex comprises DNA and a recombinase.

20. Use of a pseudocapsid as claimed in Claim 19 wherein the DNA includes a sequence of a site in the genome of the host cell.

21. Use as claimed in any one of Claims 1 to 20 wherein the pseudocapsid composition is adapted for intranasal or intraperitoneal administration to a mammal.

22. A method of making a pseudocapsid composition comprising providing an empty pseudocapsid formed from papovavirus major capsid antigen and excluding minor capsid antigens; providing exogenous material; and mixing the empty pseudocapsid and exogenous material whereby the exogenous material becomes associated with the empty pseudocapsid; wherein the exogenous material is selected so that, in use, the biological functioning of the exogenous material in a neuronal host cell has a beneficial effect on the cell or a mammal containing that cell by treating and/or preventing a neurological disease, provided that the pseudocapsid is not one formed from at least one semi-purified or pure SV40 capsid protein and a constituent selected from the group consisting of an exogenous DNA encoding an exogenous protein or peptide product, or encoding a therapeutic RNA, or itself a therapeutic product, a vector comprising exogenous DNA encoding an exogenous protein or peptide product or encoding a therapeutic RNA, or itself a therapeutic product, an exogenous RNA encoding an exogenous protein or peptide product or itself a therapeutic product, a vector comprising an exogenous RNA encoding an exogenous protein or peptide product, or itself a therapeutic product, an exogenous protein or peptide product, and antisense RNA, ribozyme RNA or any RNA or DNA which inhibits or prevents the expression of undesired protein/s in a mammalian cell, and optionally further comprising operatively linked regulatory elements sufficient for the expression and/or replication of said exogenous therapeutic RNA or of an exogenous protein or peptide in a mammalian cell.

23. A method as claimed in Claim 22 wherein the pseudocapsid composition is provided by packaging the material inside an empty pseudocapsid.

24. A method as claimed in Claim 23 wherein the material is associated with the pseudocapsid by attaching it to the pseudocapsid.

25. A method as claimed in Claim 22 wherein the mixture of empty pseudocapsid and exogenous material is subjected to osmotic shock.

26. A method as claimed in Claim 22 wherein the empty pseudocapsid and exogenous material are mixed in the presence of calcium ions.

27. A pharmaceutical composition comprising a pseudocapsid formed from a papovavirus major capsid antigen and excluding minor capsid antigens, which pseudocapsid has a pharmacologically active compound associated therewith; the pseudocapsid being provided together with a pharmacologically acceptable carrier, wherein the pharmacologically active compound is selected to have a beneficial effect on a neuronal host cell or a mammal containing the cell by treating and/or preventing a neurological disease, provided that the pseudocapsid is not one formed from at least one semi-purified or pure SV40 capsid protein and a constituent selected from the group consisting of an exogenous DNA encoding an exogenous protein or peptide product, or encoding a therapeutic RNA, or itself a therapeutic product, a vector comprising exogenous DNA encoding an exogenous protein or peptide product, or encoding a therapeutic RNA, or itself a therapeutic product, an exogenous RNA encoding an exogenous protein or peptide product or itself a therapeutic product, a vector comprising an exogenous RNA encoding an exogenous protein or peptide product, or itself a therapeutic product, an exogenous protein or peptide product, and antisense RNA, ribozyme RNA or any RNA or DNA which inhibits or prevents the expression of undesired protein/s in a mammalian cell, and optionally further comprising operatively linked regulatory elements sufficient for the expression and/or replication of said exogenous therapeutic RNA or of an exogenous protein or peptide in a mammalian cell.

28. A pharmaceutical composition as claimed in Claim 27 adapted for intranasal or intraperitoneal administration.

29. A pharmaceutical composition comprising a pseudocapsid formed from a papovavirus major capsid antigen and excluding minor capsid antigens, which pseudocapsid has a pharmacologically active compound associated therewith; the pseudocapsid being provided together with a pharmacologically acceptable carrier, wherein the pharmacologically active compound is selected to have a beneficial effect on a neuronal host cell or a mammal containing the cell by treating and/or preventing a neurological disease, and wherein the composition is adapted for intranasal or intraperitoneal administration.

30. A pharmaceutical composition as claimed in Claim 27 wherein the pharmacologically active compound acts in the nucleus of the host cell.

**Patentansprüche**

1. Verwendung eines Pseudocapsids, das aus dem Haupt-Capsidantigen des Papovavirus gebildet wird und keine Neben-Capsidantigene enthält, wobei das Pseudocapsid mit diesem assoziiertes exogenes Material aufweist, bei der Herstellung einer Pseudocapsid-Zusammensetzung zur Behandlung einer neuronalen Wirtszelle, so dass das exogene Material von der Zelle aufgenommen wird und in dieser Zelle biologisch funktionell aktiv ist, so dass eine vorteilhafte Wirkung in der Zelle oder einem Säugetier, das die Zelle enthält, hervorgerufen wird, zur Behandlung und/oder Verhinderung einer neurologischen Erkrankung; oder zur Erkennung einer neuronalen Zelle, mit der Maßgabe, dass das Pseudocapsid nicht eines ist, das aus wenigstens einem semi-gereinigten oder reinen SV40-Capsidprotein und einem Bestandteil besteht, der aus der Gruppe ausgewählt ist, die besteht aus einer exogenen DNA, die für ein exogenes Protein- oder Peptidprodukt codiert, oder die für eine therapeutische RNA codiert oder selbst ein therapeutisches Produkt ist, einem Vektor, der eine exogene DNA umfasst, die für ein exogenes Protein- oder Peptidprodukt codiert, oder der für eine therapeutische RNA codiert oder selbst ein therapeutisches Produkt ist, einer exogenen RNA, die für ein exogenes Protein- oder Peptidprodukt codiert oder selbst ein therapeutisches Produkt ist, einem Vektor, der eine exogene RNA umfasst, die für ein exogenes Protein- oder Peptidprodukt codiert oder selbst ein therapeutisches Produkt ist, einem exogenen Protein- oder Peptidprodukt und einer Antisense-RNA, einer Ribozym-RNA oder einer beliebigen RNA oder DNA, die die Expression des unerwünschten Proteins bzw. der unerwünschten Proteine in einer Säugerzelle hemmt oder verhindert, und die weiterhin gegebenenfalls funktionsfähig verknüpfte regulatorische Elemente aufweist, die für die Expression und/oder Replikation der genannten exogenen therapeutischen RNA oder eines exogenen Proteins oder Peptids in einer Säugerzelle ausreichend sind.

2. Verwendung eines Pseudocapsids, das aus dem Haupt-Capsidantigen des Papovavirus gebildet wird und keine Neben-Capsidantigene enthält, wobei das Pseudocapsid mit diesem assoziiertes exogenes Material aufweist, bei der Herstellung einer Pseudocapsid-Zusammensetzung zur Behandlung einer neuronalen Wirtszelle, so dass das exogene Material von der Zelle aufgenommen wird und in dieser Zelle biologisch funktionell aktiv ist, so dass eine vorteilhafte Wirkung in der Zelle oder einem Säugetier, das die Zelle enthält, hervorgerufen wird, zur Behandlung und/oder Verhinderung einer neurologischen Erkrankung; oder zur Erkennung einer neuronalen Zelle, wobei die neuronale Wirtszelle aus dem Zentrainervensystem eines Säugetiers stammt oder in diesem vorliegt.

3. Verwendung, wie sie im Anspruch 1 oder 2 beansprucht wird, wobei die neuronale Wirtszelle aus dem Gehirn eines Säugetiers stammt oder in diesem vorliegt.

4. Verwendung, wie sie im Anspruch 1 beansprucht wird, wobei das Haupt-Capsidantigen von einem oder mehreren aus der Gruppe stammt, die besteht aus dem humanen Papillomvirus, dem Maus-Polyomavirus, dem Rinder-Papillomvirus und dem humanen Polyomavirus BK.

5. Verwendung, wie sie im Anspruch 2 beansprucht wird, wobei das Haupt-Capsidantigen von einem oder mehreren aus der Gruppe stammt, die besteht aus dem humanen Papillomvirus, dem Maus-Polyomavirus, dem Rinder-Papillomvirus, dem Simian-40-Virus und dem humanen Polyomavirus BK.

6. Verwendung, wie sie im Anspruch 1 oder 2 beansprucht wird, wobei das Haupt-Capsidantigen VP1 des Maus-Polyomavirus ist.

**7.** Verwendung, wie sie in einem beliebigen vorhergehenden Anspruch beansprucht wird, wobei die neurologische Erkrankung aus der Alzheimer-Krankheit oder der Parkinson-Krankheit ausgewählt ist.

**8.** Verwendung, wie sie in einem beliebigen der Ansprüche 1 bis 7 beansprucht wird, wobei die Pseudocapsid-Zusammensetzung einen pharmazeutisch annehmbaren Träger aufweist.

**9.** Verwendung, wie sie in einem beliebigen der Ansprüche 1 bis 8 beansprucht wird, wobei das exogene Material genetisches Material ist.

**10.** Verwendung eines Pseudocapsids, wie sie im Anspruch 9 beansprucht wird, wobei das genetische Material alles oder einen codierenden Teil von wenigstens einem Gen umfasst.

**11.** Verwendung, wie sie im Anspruch 10 beansprucht wird, wobei das Gen aus einem oder mehreren der Gruppe ausgewählt ist, die besteht aus dem *nerve growth factor,* der Tyrosinhydroxylase oder dem die neurale Apoptose hemmenden Protein.

**12.** Verwendung, wie sie im Anspruch 10 oder 11 beansprucht wird, wobei sich die Expression des Gens oder der Gene unter der Kontrolle eines für das Nervensystem spezifischen Promotors oder eines Promotors, der für eine spezielle Subpopulation neuronaler Zellen spezifisch ist, befindet.

**13.** Verwendung, wie sie im Anspruch 12 beansprucht wird, wobei der Promotor ausgewählt ist aus den Promotoren für die leichte Neurofilament-Kette, die neuronenspezifische Enolase, die Tyrosinhydroxylase, die Dopamin-β-Hydroxylase, das saure fibrilläre Gliaprotein, das Tα1-Tubulin, die β-Kette des *platelet-derived growth factor,* das basische Myelinprotein und das Präproenkaphlin.

**14.** Verwendung eines Pseudocapsids, wie sie in einem beliebigen der Ansprüche 1 bis 8 beansprucht wird, wobei das exogene Material ein Protein oder ein Polypeptid ist.

**15.** Verwendung eines Pseudocapsids, wie sie in einem beliebigen der Ansprüche 1 bis 8 beansprucht wird, wobei das Protein oder Polypeptid ein Antikörper oder ein Fragment davon ist.

**16.** Verwendung eines Pseudocapsids, wie sie in einem beliebigen der Ansprüche 1 bis 8 beansprucht wird, wobei das exogene Material eine pharmakologisch aktive Verbindung ist.

**17.** Verwendung eines Pseudocapsids, wie sie in einem beliebigen der Ansprüche 1 bis 8 beansprucht wird, wobei das exogene Material eine Peptid-Nucleinsäure ist.

**18.** Verwendung eines Pseudocapsids, wie sie in einem beliebigen der Ansprüche 1 bis 8 beansprucht wird, wobei das exogene Material einen Komplex aus biologischen Makromolekülen umfasst.

**19.** Verwendung eines Pseudocapsids, wie sie im Anspruch 18 beansprucht wird, wobei der Komplex DNA und eine Rekombinase umfasst.

**20.** Verwendung eines Pseudocapsids, wie sie im Anspruch 19 beansprucht wird, wobei die DNA eine Sequenz eines Abschnitts im Genom der Wirtszelle einschließt.

**21.** Verwendung, wie sie in einem beliebigen der Ansprüche 1 bis 20 beansprucht wird, wobei die Pseudocapsid-Zusammensetzung an eine intranasale oder intraperitoneale Verabreichung an ein Säugetier adaptiert ist.

**22.** Verfahren zur Herstellung einer Pseudocapsid-Zusammensetzung, das umfasst das Bereitstellen eines leeren Pseudocapsids, das aus dem Haupt-Capsidantigen des Papovavirus gebildet wird und keine Neben-Capsidantigene enthält, das Bereitstellen eines exogenen Materials und das Mischen des leeren Pseudocapsids und des exogenen Materials, wodurch das exogene Material mit dem leeren Pseudocapsid assoziiert wird; wobei das exogene Material so ausgewählt ist, dass bei der Anwendung die Ausübung der biologischen Funktion des exogenen Materials in einer neuronalen Wirtszelle eine vorteilhafte Wirkung auf die Zelle oder ein Säugetier, das die Zelle enthält, hat, indem eine neurologische Erkrankung behandelt und/oder verhindert wird, mit der Maßgabe, dass das Pseudocapsid nicht eines ist, das aus wenigstens einem semi-gereinigten oder reinen SV40-Capsidprotein und einem Bestandteil besteht, der aus der Gruppe ausgewählt ist, die besteht aus einer exogenen DNA, die für ein exogenes Protein- oder Peptidprodukt codiert, oder die für eine therapeutische RNA codiert oder selbst ein therapeutisches Produkt ist, einem Vektor, der eine exogene DNA umfasst, die für ein exogenes Protein- oder Peptidprodukt codiert, oder der für eine therapeutische RNA codiert oder selbst ein therapeutisches Produkt ist, einer exogenen RNA, die für ein exogenes Protein- oder Peptidprodukt codiert oder selbst ein therapeutisches Produkt ist, einem Vektor, der eine exogene RNA umfasst, die für ein exogenes Protein- oder Peptidprodukt codiert oder selbst ein therapeutisches Produkt ist, einem exogenen Protein- oder Peptidprodukt und einer Antisense-RNA, einer Ribozym-RNA oder einer beliebigen RNA oder DNA, die die Expression des unerwünschten Proteins bzw der unerwünschten Proteine in einer Säugerzelle hemmt oder verhindert, und der gegebenenfalls weiterhin funktionsfähig verknüpfte regulatorische Elemente aufweist, die für die Expression und/oder Replikation der genannten exogenen therapeutischen RNA oder eines exogenen Proteins oder Peptids in einer Säugerzelle ausreichend sind.

23. Verfahren, wie es im Anspruch 22 beansprucht wird, wobei die Pseudocapsid-Zusammensetzung durch das Verpacken des Materials im Inneren eines leeren Pseudocapsids bereit gestellt wird.

24. Verfahren, wie es im Anspruch 23 beansprucht wird, wobei das Material über seine Befestigung am Pseudocapsid mit dem Pseudocapsid assoziiert ist.

25. Verfahren, wie es im Anspruch 22 beansprucht wird, wobei die Mischung aus dem leeren Pseudocapsid und dem exogenen Material einem osmotischen Schock unterzogen wird.

26. Verfahren, wie es im Anspruch 22 beansprucht wird, wobei das leere Pseudocapsid und das exogene Material in Gegenwart von Calciumionen gemischt werden.

27. Pharmazeutische Zusammensetzung, die ein Pseudocapsid aufweist, das aus dem Haupt-Capsidantigen des Papovavirus gebildet wird und keine Neben-Capsidantigene enthält, wobei das Pseudocapsid eine mit ihm assoziierte pharmakologisch aktive Verbindung aufweist; wobei das Pseudocapsid zusammen mit einem pharmakologisch annehmbaren Träger bereit gestellt wird, wobei die pharmakologisch aktive Verbindung so ausgewählt ist, dass sie eine vorteilhafte Wirkung auf eine neuronale Wirtszelle oder ein Säugetier, das die Zelle enthält, ausübt, indem sie eine neurologische Erkrankung behandelt und/oder verhindert, mit der Maßgabe, dass das Pseudocapsid nicht eines ist, das aus wenigstens einem semi-gereinigten oder reinen SV40-Capsidprotein und einem Bestandteil besteht, der aus der Gruppe ausgewählt ist, die besteht aus einer exogenen DNA, die für ein exogenes Protein- oder Peptidprodukt codiert, oder die für eine therapeutische RNA codiert oder selbst ein therapeutisches Produkt ist, einem Vektor, der eine exogene DNA umfasst, die für ein exogenes Protein- oder Peptidprodukt codiert, oder der für eine therapeutische RNA codiert oder selbst ein therapeutisches Produkt ist, einer exogenen RNA, die für ein exogenes Protein- oder Peptidprodukt codiert oder selbst ein therapeutisches Produkt ist, einem Vektor, der eine exogene RNA umfasst, die für ein exogenes Protein- oder Peptidprodukt codiert oder selbst ein therapeutisches Produkt ist, einem exogenen Protein- oder Peptidprodukt und einer Antisense-RNA, einer Ribozym-RNA oder einer beliebigen RNA oder DNA, die die Expression des unerwünschten Proteins bzw. der unerwünschten Proteine in einer Säugerzelle hemmt oder verhindert, und der gegebenenfalls weiterhin funktionsfähig verknüpfte regulatorische Elemente aufweist, die für die Expression und/oder Replikation der genannten exogenen therapeutischen RNA oder eines exogenen Proteins oder Peptids in einer Säugerzelle ausreichend sind.

28. Pharmazeutische Zusammensetzung, wie sie im Anspruch 27 beansprucht wird, die an eine intranasale oder intraperitoneale Verabreichung adaptiert ist.

29. Pharmazeutische Zusammensetzung, die ein Pseudocapsid aufweist, das aus dem Haupt-Capsidantigen des Papovavirus gebildet wird und keine Neben-Capsidantigene enthält, wobei das Pseudocapsid eine mit ihm assoziierte pharmakologisch aktive Verbindung aufweist; wobei das Pseudocapsid zusammen mit einem pharmakologisch annehmbaren Träger bereit gestellt wird, wobei die pharmakologisch aktive Verbindung so ausgewählt ist, dass sie eine vorteilhafte Wirkung auf eine neuronale Wirtszelle oder ein Säugetier, das die Zelle enthält, ausübt, indem sie eine neurologische Erkrankung behandelt und/oder verhindert, und wobei die Zusammensetzung an eine intranasale oder intraperitoneale Verabreichung adaptiert ist.

30. Pharmazeutische Zusammensetzung, wie sie im Anspruch 27 beansprucht wird, wobei die pharmakologisch aktive Verbindung im Kem der Wirtszelle wirkt.

**EP 0 977 596 B1**

**Revendications**

1. Utilisation d'une pseudocapside formée à partir d'un antigène principal de capside d'un virus du groupe Papova et excluant les antigènes mineurs de capside, laquelle pseudocapside a un matériel exogène associé à celle-ci, dans la production d'une composition d'une pseudocapside pour le traitement d'une cellule nerveuse hôte de manière à ce que le matériel exogène soit pris par la cellule et qu'il soit biologiquement fonctionnel dans cette même cellule pour produire un effet bénéfique dans la cellule ou chez un mammifère comprenant ladite cellule, pour le traitement et/ou la prévention d'une maladie neurologique ; ou pour la détection d'une cellule nerveuse, à condition que la pseudocapside ne soit pas une pseudocapside formée à partir d'au moins une protéine de capside SV40 semi-purifiée ou pure et d'un constituant choisi dans le groupe constitué d'un ADN exogène codant pour une protéine ou un produit peptidique exogène, ou codant pour un ARN thérapeutique, ou lui-même un produit thérapeutique, un vecteur comprenant un ADN exogène codant pour une protéine ou un produit peptidique exogène, ou codant pour un ARN thérapeutique, ou lui-même un produit thérapeutique, un ARN exogène codant pour une protéine ou un produit peptidique exogène ou lui-même un produit thérapeutique, un vecteur comprenant un ARN exogène codant pour une protéine ou un produit peptidique exogène, ou lui-même un produit thérapeutique, une protéine ou un produit peptidique exogène, et un ARN antisens, un ARN ribosomal ou un ARN ou ADN quelconque qui inhibe ou empêche l'expression d'une ou plusieurs protéine(s) non désirée(s) dans une cellule de mammifère, et comprenant éventuellement en outre des éléments régulateurs liés d'une façon fonctionnelle suffisants pour l'expression et/ou la réplication dudit ARN thérapeutique exogène ou d'une protéine ou d'un peptide exogène dans une cellule de mammifère.

2. Utilisation d'une pseudocapside formée à partir d'un antigène principal de capside d'un virus du groupe Papova et excluant les antigènes mineurs de capside, laquelle pseudocapside a un matériel exogène associé à celle-ci, dans la production d'une composition d'une pseudocapside pour le traitement d'une cellule nerveuse hôte de manière à ce que le matériel exogène soit pris par la cellule et qu'il soit biologiquement fonctionnel dans cette même cellule, pour produire un effet bénéfique dans la cellule ou chez un mammifère comprenant ladite cellule, pour le traitement et/ou la prévention d'une maladie neurologique ; ou pour la détection d'une cellule nerveuse, dans laquelle la cellule nerveuse hôte est dérivée à partir du ou est présente dans le système nerveux central d'un mammifère.

3. Utilisation selon la revendication 1 ou la revendication 2, dans laquelle la cellule nerveuse hôte est dérivée à partir du ou est présente dans le cerveau d'un mammifère.

4. Utilisation selon la revendication 1, dans laquelle l'antigène principal de capside provient d'un ou plusieurs virus du papillome humain, virus du polyome de souris, virus du papillome bovin ou du virus BK du polyome humain.

5. Utilisation selon la revendication 2, dans laquelle l'antigène principal de capside provient d'un ou plusieurs virus du papillome humain, virus du polyome de souris, virus du papillome bovin, virus simien 40 ou du virus BK du polyome humain.

6. Utilisation selon la revendication 1 ou la revendication 2, dans laquelle l'antigène principal de capside est VP1 issu du virus du polyome de souris.

7. Utilisation selon l'une quelconque des revendications précédentes, dans laquelle la maladie neurologique est choisie entre la maladie d'Alzheimer et la maladie de Parkinson.

8. Utilisation selon l'une quelconque des revendications 1 à 7, dans laquelle la composition d'une pseudocapside comprend un véhicule acceptable sur le plan pharmacologique.

9. Utilisation selon l'une quelconque des revendications 1 à 8, dans laquelle le matériel exogène est un matériel génétique.

10. Utilisation d'une pseudocapside selon la revendication 9, dans laquelle le matériel génétique comprend l'intégralité ou une partie codante d'au moins un gène.

11. Utilisation selon la revendication 10, dans laquelle le gène est choisi dans l'un ou plusieurs parmi un facteur de croissance du tissu nerveux, la tyrosine hydroxylase ou la protéine inhibitrice de l'apoptose des neurones.

**12.** Utilisation selon la revendication 10 ou la revendication 11, dans laquelle l'expression du gène ou des gènes se trouve sous le contrôle d'un promoteur spécifique au système nerveux ou d'un promoteur spécifique à une sous-population de neurones particuliers.

**13.** Utilisation selon la revendication 12, dans laquelle le promoteur est choisi parmi les promoteurs correspondants à une chaîne légère de neurofilaments, l'énolase spécifique aux neurones, la tyrosine hydroxylase, la dopamine β-hydroxylase, la protéine fibrillaire acide des cellules gliales, la tubuline Tα1, la chaîne β du facteur de croissance dérivé des plaquettes, la protéine basique myéline et la préproencéphaline.

**14.** Utilisation d'une pseudocapside selon l'une quelconque des revendications 1 à 8, dans laquelle le matériel exogène est une protéine ou un polypeptide.

**15.** Utilisation d'une pseudocapside selon l'une quelconque des revendications 1 à 8, dans laquelle la protéine ou le polypeptide est un anticorps ou un fragment de celui-ci.

**16.** Utilisation d'une pseudocapside selon l'une quelconque des revendications 1 à 8, dans laquelle le matériel exogène est un composé actif sur le plan pharmacologique.

**17.** Utilisation d'une pseudocapside selon l'une quelconque des revendications 1 à 8, dans laquelle le matériel exogène est un acide nucléique peptidique.

**18.** Utilisation d'une pseudocapside selon l'une quelconque des revendications 1 à 8, dans laquelle le matériel exogène comprend un complexe de macromolécules biologiques.

**19.** Utilisation d'une pseudocapside selon la revendication 18, dans laquelle le complexe comprend un ADN et une recombinase.

**20.** Utilisation d'une pseudocapside selon la revendication 19, dans laquelle l'ADN comprend une séquence d'un site dans le génome de la cellule hôte.

**21.** Utilisation selon l'une quelconque des revendications 1 à 20, dans laquelle la composition d'une pseudocapside est adaptée à l'administration par voie intranasale ou intrapéritonéale à un mammifère.

**22.** Méthode de préparation d'une composition d'une pseudocapside comprenant la fourniture d'une pseudocapside vide formée à partir d'un antigène principal de capside d'un virus du groupe Papova et excluant les antigènes mineurs de capside ; la fourniture d'un matériel exogène ; puis le mélange de la pseudocapside vide et du matériel exogène de manière à ce que le matériel exogène soit associé à la pseudocapside vide ; dans laquelle le matériel exogène est choisi de manière à ce que, à l'utilisation, le fonctionnement biologique du matériel exogène dans une cellule nerveuse hôte produise un effet bénéfique dans la cellule ou chez un mammifère comprenant ladite cellule par le traitement et/ou la prévention d'une maladie neurologique, à condition que la pseudocapside ne soit pas une pseudocapside formée à partir d'au moins une protéine de capside SV40 semi-purifiée ou pure et d'un constituant choisi dans le groupe constitué d'un ADN exogène codant pour une protéine ou un produit peptidique exogène, ou codant pour un ARN thérapeutique, ou lui-même un produit thérapeutique, un vecteur comprenant un ADN exogène codant pour une protéine ou un produit peptidique exogène, ou codant pour un ARN thérapeutique, ou lui-même un produit thérapeutique, un ARN exogène codant pour une protéine ou un produit peptidique exogène ou lui-même un produit thérapeutique, un vecteur comprenant un ARN exogène codant pour une protéine ou un produit peptidique exogène, ou lui-même un produit thérapeutique, une protéine ou un produit peptidique exogène, et un ARN antisens, un ARN ribosomal ou un ARN ou ADN quelconque qui inhibe ou empêche l'expression d'une ou plusieurs protéine(s) non désirée(s) dans une cellule de mammifère, et comprenant éventuellement en outre des éléments régulateurs liés d'une façon fonctionnelle suffisants pour l'expression et/ou la réplication dudit ARN thérapeutique exogène ou d'une protéine ou d'un peptide exogène dans une cellule de mammifère.

**23.** Méthode selon la revendication 22, dans laquelle la composition d'une pseudocapside est fournie en emballant le matériel à l'intérieur d'une pseudocapside vide.

**24.** Méthode selon la revendication 23, dans laquelle le matériel est associé à la pseudocapside en l'attachant à la pseudocapside.

**25.** Méthode selon la revendication 22, dans laquelle le mélange de pseudocapside vide et de matériel exogène est soumis à un choc osmotique.

**26.** Méthode selon la revendication 22, dans laquelle la pseudocapside vide et le matériel exogène sont mélangés en présence d'ions calcium.

**27.** Composition pharmaceutique comprenant une pseudocapside formée à partir d'un antigène principal de capside d'un virus du groupe Papova et excluant les antigènes mineurs de capside, laquelle pseudocapside a un composé actif sur le plan pharmacologique associé à celle-ci ; la pseudocapside étant fournie conjointement à un véhicule acceptable sur le plan pharmacologique, dans laquelle le composé actif sur le plan pharmacologique est choisi de manière à avoir un effet bénéfique dans la cellule nerveuse hôte ou chez un mammifère comprenant ladite cellule par le traitement et/ou la prévention d'une maladie neurologique, à condition que la pseudocapside ne soit pas une pseudocapside formée à partir d'au moins une protéine de capside SV40 semi-purifiée ou pure et d'un constituant choisi dans le groupe constitué d'un ADN exogène codant pour une protéine ou un produit peptidique exogène, ou codant pour un ARN thérapeutique, ou lui-même un produit thérapeutique, un vecteur comprenant un ADN exogène codant pour une protéine ou un produit peptidique exogène, ou codant pour un ARN thérapeutique, ou lui-même un produit thérapeutique, un ARN exogène codant pour une protéine ou un produit peptidique exogène ou lui-même un produit thérapeutique, un vecteur comprenant un ARN exogène codant pour une protéine ou un produit peptidique exogène, ou lui-même un produit thérapeutique, une protéine ou un produit peptidique exogène, et un ARN antisens, un ARN ribosomal ou un ARN ou ADN quelconque qui inhibe ou empêche l'expression d'une ou plusieurs protéine(s) non désirée(s) dans une cellule de mammifère, et comprenant éventuellement en outre des éléments régulateurs liés d'une façon fonctionnelle suffisants pour l'expression et/ou la réplication dudit ARN thérapeutique exogène ou d'une protéine ou d'un peptide exogène dans une cellule de mammifère.

**28.** Composition pharmaceutique selon la revendication 27, adaptée à l'administration par voie intranasale ou intrapéritonéale.

**29.** Composition pharmaceutique comprenant une pseudocapside formée à partir d'un antigène principal de capside d'un virus du groupe Papova et excluant les antigènes mineurs de capside, laquelle pseudocapside a un composé actif sur le plan pharmacologique associé à celle-ci ; la pseudocapside étant fournie conjointement à un véhicule acceptable sur le plan pharmacologique, dans laquelle le composé actif sur le plan pharmacologique est choisi de manière à avoir un effet bénéfique sur une cellule nerveuse hôte ou chez un mammifère comprenant ladite cellule par le traitement et/ou la prévention d'une maladie neurologique, et dans laquelle la composition est adaptée à l'administration par voie intranasale ou intrapéritonéale.

**30.** Composition pharmaceutique selon la revendication 27, dans laquelle le composé actif sur le plan pharmacologique agit dans le noyau de la cellule hôte.

Fig. 1(A)

Fig. 1(B)

Fig. 1(C)

Fig. 2(a)

Fig. 2(b)

Fig. 2(c)

Fig. 2(d)

EP 0 977 596 B1

1  2   3  4 5

— —— *dl8* LT

—— — —— — —— *dl8* MT

# Fig. 3(A)

1      2      3      4      5
C M    CM     CM     CM     CM

—97

—68

— — ≡ ═ —— *dl8* MT
—43

# Fig. 3(B)

Fig. 4

1 2 3 4 5 6

p43 ► ● ─ ─ ● ─  *Fig. 5(A)*

1 2 3 4 5 6

tubulin ► ●━━━━●

*Fig. 5(B)*

*Eco* RI
1560
stop

*d18*
990-1080

MT intron
deletion
747-808

*d18* MT
gene

pPyMT*d18*

ATG
174

ori

*Bcl* I
5021

*Bam* HI
4632

pAT 153

*Bam* HI
4632

*Fig. 6*

100nm

*Fig. 7*

Fig. 8

*Fig. 8(D)*

Fig. 9A

Fig. 9B

*Fig. 10*

*Fig. 11*

**Fig. 12**

Fig. 13

*(a)*

*(b)*

*(c)*

*(d)*

*Fig. 14*

*(A)*                                          *(B)*

Fig. 15

EP 0 977 596 B1

*Fig. 16*

55

*(a)*

*(b)*

# Fig. 17(A)

*Fig. 17(B)*

*Fig. 17(C)*